(19) Europäisches Patentamt
European Patent Office
Office européen des brevets

(11) Publication number: **0 013 145**
**A2**

(12) # EUROPEAN PATENT APPLICATION

(21) Application number: 79302988.5

(22) Date of filing: 20.12.79

(51) Int. Cl.³: A 01 N 43/40
C 07 D 213/74

(30) Priority: 22.12.78 AU 7202/78
19.03.79 AU 8093/79

(43) Date of publication of application:
09.07.80 Bulletin 80/14

(84) Designated Contracting States:
BE CH DE FR GB IT NL

(71) Applicant: ICI AUSTRALIA LIMITED
ICI House 1 Nicholson Street P.O.Box 4311
Melbourne Victoria 3001(AU)

(72) Inventor: Serban, Alexander
3 Maple Court
Doncaster, Victoria 3108(AU)

(72) Inventor: Watson, Keith Geoffrey
36 Medway Street
Box Hill North, Victoria 3129(AU)

(72) Inventor: Bird, Graham John
1 Burrindi Road
South Caulfield, Victoria 3162(AU)

(74) Representative: Fawcett, Richard Fennelly et al.
Imperial Chemical Industries Limited Legal Department:
Patents Thames House North Millbank
London SW1P 4QG(GB)

(54) **Substituted anilinopyridines, processes for their preparation and intermediates for use therein, compositions and herbicidal use thereof.**

(57) The invention concerns novel compounds of the formula I

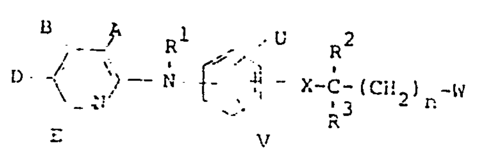

I

The compounds are herbicides and in further embodiments the invention provides herbicidal compositions containing as active ingredient a compound of formula I, a process for severely damaging or killing unwanted plants by applying to the plants or to the growth medium of the plants an effective amount of a compound of formula I, processes for the preparation of compounds of formula I and intermediates useful in the preparation of compounds of formula I.

EP 0 013 145 A2



INCOMPLETE DOCUMENT

This invention relates to organic compounds having biological activity and in particular to organic compounds having herbicidal properties, to processes for the preparation of such compounds, to intermediates useful in the preparation of such compounds and to herbicidal compositions and processes utilizing such compounds.

We have now found a new class of pyridines which exhibit biological activity, and in particular herbicidal activity.

Accordingly the invention provides a compound of formula I:

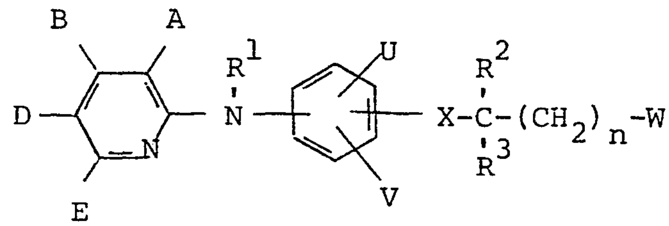

I

or a salt thereof wherein:

A, B, D, E, U and V are independently chosen from the group consisting of hydrogen, halogen, nitro, cyano, thiocyano, amino, $C_1$ to $C_6$ alkylamino, di($C_1$ to $C_6$ alkyl)amino, $C_1$ to $C_6$ alkyl, $C_1$ to $C_6$ haloalkyl, $C_2$ to $C_6$ alkenyl, $C_3$ to $C_7$ cycloalkyl, $C_1$ to $C_6$ alkoxy, $C_1$ to $C_6$ haloalkoxy, $C_1$ to $C_6$ alkylthio, $C_1$ to $C_6$ alkylsulfinyl, $C_1$ to $C_6$ alkylsulfonyl, $C_1$ to $C_6$ haloalkylsulfinyl, $C_1$ to $C_6$ haloalkylsulfonyl, sulfo, $C_1$ to $C_6$ alkoxysulfonyl, sulfamoyl, N-($C_1$ to $C_6$ alkyl)sulfamoyl, N,N-di($C_1$ to $C_6$ alkyl)sulfamoyl, carboxy, ($C_1$ to $C_6$ alkoxy)carbonyl, carbamoyl, N-($C_1$ to $C_6$ alkyl)carbamoyl, N,N-di($C_1$ to $C_6$ alkyl)carbamoyl, phenyl, phenoxy, phenylthio, and the groups substituted phenyl, substituted phenoxy and substituted phenylthio wherein in each group the phenyl ring is substituted with one to three substituents chosen from the group consisting of halogen, $C_1$ to $C_6$ alkyl, $C_1$ to $C_6$ haloalkyl, $C_1$ to $C_6$ alkoxy, nitro and cyano;

$R^1$ is chosen from the group consisting of hydrogen, $C_1$ to $C_{10}$ alkyl, $C_2$ to $C_{10}$ alkenyl, $C_2$ to $C_{10}$ alkynyl, $C_2$ to $C_{10}$ alkoxyalkyl, $C_1$ to $C_{10}$ haloalkyl, formyl, $C_2$ to $C_{10}$ alkanoyl, phenyl, benzyl, benzoyl, and the groups phenyl, benzyl and benzoyl wherein in each group the phenyl ring is substituted with one or two substituents chosen from the group consisting of halogen, $C_1$ to $C_6$ alkyl, $C_1$ to $C_6$ haloalkyl, $C_1$ to $C_6$ alkoxy, nitro and cyano;

$R^2$ is chosen from the group consisting of hydrogen, $C_1$ to $C_6$ alkyl, $C_2$ to $C_6$ alkenyl, $C_2$ to $C_6$ alkoxyalkyl, $C_1$ to $C_6$ haloalkyl, acetyl, propionyl, and $C_2$ to $C_6$ alkoxycarbonyl;

$R^3$ is chosen from the group consisting of hydrogen, $C_1$ to $C_6$ alkyl, $C_2$ to $C_6$ alkenyl, $C_2$ to $C_6$ alkoxyalkyl and $C_1$ to $C_6$ haloalkyl, or $R^2$ and $R^3$ together may form a methylene, ethylidene, propylidene or isopropylidene group;

W is chosen from the group consisting of cyano, thiocarbamoyl, $-\overset{O}{\overset{\|}{C}}-G$ and $CH_2Z$ wherein:

G is chosen from the group consisting of hydroxy, mercapto, $C_1$ to $C_{10}$ alkoxy, $C_1$ to $C_{10}$ haloalkoxy, $C_2$ to $C_{10}$ alkenyloxy, $C_2$ to $C_{10}$ alkynyloxy, $C_1$ to $C_{10}$ alkylthio, $C_2$ to $C_{10}$ alkenylthio, $C_2$ to $C_{10}$ alkynylthio, $C_3$ to $C_7$ cycloalkoxy, $C_3$ to $C_7$ cycloalkoxy substituted with one or two $C_1$ to $C_4$ alkyl groups, phenoxy, phenylthio, benzyloxy, benzylthio, the group $C_1$ to $C_{10}$ alkoxy substituted with a substituent chosen from the group consisting of $C_1$ to $C_6$ alkoxy, amino, ammonio, cyano, N-($C_1$ to $C_6$ alkyl)amino, N,N-di($C_1$ to $C_6$ alkyl)-amino and N,N,N-tri($C_1$ to $C_6$ alkyl)ammonio, the groups phenoxy, phenylthio, benzyloxy and benzylthio wherein in each group the phenyl ring is substituted with one or two substituents chosen from the group consisting of halogen, nitro, cyano, $C_1$ to $C_6$ alkyl, $C_1$ to $C_6$ halo-

alkyl and $C_1$ to $C_6$ alkoxy, the group OM wherein M is the cation of an inorganic or organic base, the group $-HNSO_2R^4$ wherein $R^4$ is chosen from $C_1$ to $C_{10}$ alkyl and $C_1$ to $C_{10}$ haloalkyl, and the group $-NR^5R^6$ wherein $R^5$ and $R^6$ are independently chosen from the group consisting of hydrogen, $C_1$ to $C_6$ alkyl, phenyl and benzyl or $R^5$ and $R^6$ together form a heterocyclic ring; and Z is chosen from halogen, hydroxy, mercapto, $C_1$ to $C_{10}$ alkoxy, $C_1$ to $C_{10}$ haloalkoxy, $C_1$ to $C_{10}$ alkylthio and the group $NR^5R^6$ wherein $R^5$ and $R^6$ are as hereinbefore defined;

X is chosen from oxygen and sulfur; and

n is 0, 1 or 2.

The compounds of formula I wherein $R^2$ and $R^3$ are not the same are optically active and the present invention also includes the individual stereo isomers of such compounds and mixtures of those stereo isomers in addition to the racemic mixture of stereo isomers.

Suitable A, B, D, E, U and V include hydrogen, halogen, nitro, cyano, thiocyano, amino, $C_1$ to $C_6$ alkylamino, di($C_1$ to $C_6$ alkyl)amino, $C_1$ to $C_6$ alkyl, $C_1$ to $C_6$ haloalkyl, $C_2$ to $C_6$ alkenyl, $C_3$ to $C_7$ cycloalkyl, $C_1$ to $C_6$ alkoxy, $C_1$ to $C_6$ alkylthio, ($C_1$ to $C_6$ alkoxy)carbonyl, phenyl, phenoxy, phenylthio and the groups substituted phenyl, substituted phenoxy and substituted phenylthio wherein in each group the phenyl ring is substituted with one to three substituents chosen from the group consisting of halogen, $C_1$ to $C_6$ alkyl, $C_1$ to $C_6$ haloalkyl, $C_1$ to $C_6$ alkoxy, nitro and cyano.

Suitable $R^1$ include hydrogen, $C_1$ to $C_{10}$ alkyl, $C_2$ to $C_{10}$ alkenyl, $C_2$ to $C_{10}$ alkoxyalkyl, $C_1$ to $C_{10}$ haloalkyl, formyl, $C_2$ to $C_{10}$ alkanoyl, phenyl, benzyl, benzoyl and the groups phenyl, benzyl and benzoyl wherein in each group the phenyl ring is substituted with one or two substitutents chosen from the group consisting of halogen, $C_1$ to $C_6$ alkyl, $C_1$ to $C_6$ haloalkyl, $C_1$ to $C_6$

alkoxy, nitro and cyano.

Suitable $R^2$ include hydrogen, $C_1$ to $C_6$ alkyl, $C_2$ to $C_6$ alkenyl, $C_2$ to $C_6$ alkoxyalkyl, $C_1$ to $C_6$ haloalkyl, acetyl, propionyl and $C_2$ to $C_6$ alkoxycarbonyl.

Suitable $R^3$ include hydrogen, $C_1$ to $C_6$ alkyl, $C_2$ to $C_6$ alkenyl, $C_2$ to $C_6$ alkoxyalkyl and $C_1$ to $C_6$ haloalkyl.

Suitable $R^2$ and $R^3$ together include methylene, ethylidene, propylidene and isopropylidene.

Suitable W include the groups cyano, thiocarbamoyl, $-\overset{O}{\overset{\|}{C}}-G$ and $-CH_2Z$.

Suitable G include hydroxy, mercapto, $C_1$ to $C_{10}$ alkoxy, $C_1$ to $C_{10}$ haloalkoxy, $C_2$ to $C_{10}$ alkenyloxy, $C_2$ to $C_{10}$ alkynyloxy, $C_1$ to $C_{10}$ alkylthio, $C_2$ to $C_{10}$ alkenylthio, $C_3$ to $C_7$ cycloalkoxy, $C_3$ to $C_7$ cycloalkoxy substituted with one or two $C_1$ to $C_4$ alkyl groups, phenoxy, phenylthio, benzyloxy, benzylthio, the group $C_1$ to $C_{10}$ alkoxy substituted with a $C_1$ to $C_6$ alkoxy group, the groups phenoxy, phenylthio, benzyloxy and benzylthio wherein in each group the phenyl ring is substituted with one or two substituents chosen from the group consisting of halogen, nitro, cyano, $C_1$ to $C_6$ alkyl, $C_1$ to $C_6$ haloalkyl and $C_1$ to $C_6$ alkoxy, the group OM wherein M is the cation of an inorganic or organic base, the group $-NHSO_2R^4$ wherein $R^4$ is chosen from $C_1$ to $C_{10}$ alkyl and $C_1$ to $C_{10}$ haloalkyl, and the group $-NR^5R^6$ wherein $R^5$ and $R^6$ are independently chosen from the group consisting of hydrogen, $C_1$ to $C_6$ alkyl, phenyl and benzyl or $R^5$ and $R^6$ together form a heterocyclic ring.

When G is the group OM suitable M include alkali metal ions, alkaline earth metal ions and the ammonium ion $H\overset{\oplus}{N}R^7R^8R^9$ wherein $R^7$, $R^8$ and $R^9$ are independently chosen from hydrogen, $C_1$ to $C_6$ alkyl, $C_2$ to $C_6$ hydroxyalkyl, phenyl and benzyl.

Suitable Z include halogen, hydroxy, mercapto,

$C_1$ to $C_{10}$ alkoxy, $C_1$ to $C_{10}$ haloalkoxy, $C_1$ to $C_{10}$ alkylthio and the group $-NR^5R^6$ wherein $R^5$ and $R^6$ are independently chosen from the group consisting of hydrogen, $C_1$ to $C_6$ alkyl, phenyl and benzyl or $R^5$ and $R^6$ together form a heterocyclic ring.

When G or Z is the group $-NR^5R^6$ wherein $R^5$ and $R^6$ together form a heterocyclic ring suitable heterocyclic rings include morpholino, piperidino, 1-piperazinyl and 1-pyrrolidinyl.

Preferred A, B, D and E include hydrogen, halogen, nitro, cyano, amino, $C_1$ to $C_6$ alkyl and $C_1$ to $C_6$ haloalkyl.

Preferred U and V include hydrogen, halogen and nitro.

Preferred $R^1$ include hydrogen, $C_1$ to $C_6$ alkyl, $C_2$ to $C_6$ alkenyl, $C_2$ to $C_6$ alkynyl, $C_2$ to $C_6$ alkanoyl and benzyl.

Preferred $R^2$ and $R^3$ include hydrogen and $C_1$ to $C_6$ alkyl.

Preferred W are the groups:

a) $-\overset{O}{\overset{\|}{C}}-G$ wherein G is chosen from hydroxy, $C_1$ to $C_{10}$ alkoxy, $C_2$ to $C_{10}$ alkenyloxy, $C_2$ to $C_{10}$ alkynyloxy, $C_3$ to $C_7$ cycloalkoxy, benzyloxy, $C_1$ to $C_{10}$ alkoxy substituted with a substituent chosen from amino, ammonio, N-($C_1$ to $C_6$ alkyl)amino, N,N-di($C_1$ to $C_6$ alkyl)amino and N,N,N-tri($C_1$ to $C_6$ alkyl)ammonio, and the group OM wherein M is an alkali metal or alkaline earth metal ion; and

b) $-CH_2Z$ wherein Z is chosen from halogen, hydroxy, mercapto and $C_1$ to $C_{10}$ alkoxy.

Preferred X is oxygen.

Preferred n is 0 or 2.

Preferred compounds of the invention are those compounds of formula II

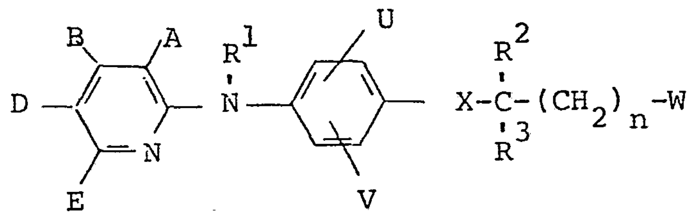

II

More preferred compounds of the invention are those in which:

A is chosen from hydrogen, halogen, nitro and $C_1$ to $C_6$ haloalkyl;

D is chosen from hydrogen, halogen, nitro, $C_1$ to $C_6$ alkyl and $C_1$ to $C_6$ haloalkyl;

$R^1$ is chosen from hydrogen, $C_1$ to $C_6$ alkyl, $C_2$ to $C_6$ alkenyl, $C_2$ to $C_6$ alkynyl and benyzl;

U is chosen from hydrogen, halogen and nitro;

$R^2$ is chosen from hydrogen and $C_1$ to $C_6$ alkyl;

B, E, V and $R^3$ are hydrogen;

X is oxygen;

W is the group $-\overset{O}{\overset{\|}{C}}-G$ wherein G is chosen from hydroxy, $C_1$ to $C_6$ alkoxy, $C_2$ to $C_6$ alkenyloxy, $C_2$ to $C_6$ alkynyloxy and $C_1$ to $C_6$ alkoxy substituted with a group chosen from N,N-di($C_1$ to $C_6$ alkyl)amino and N,N,N-tri($C_1$ to $C_6$ alkyl)-ammonio; and

n is 0.

Examples of the compounds embraced by the invention include:

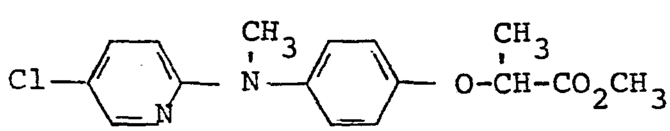

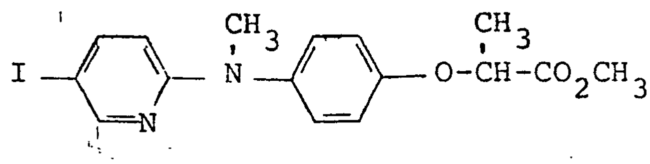

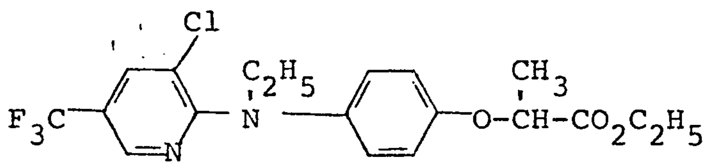

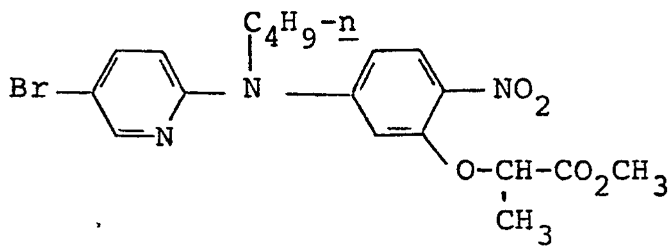

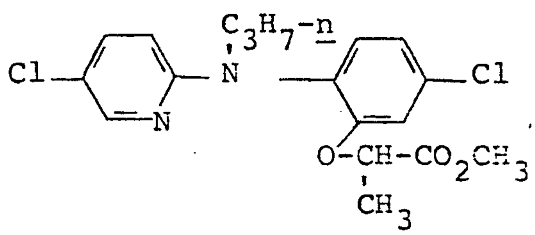

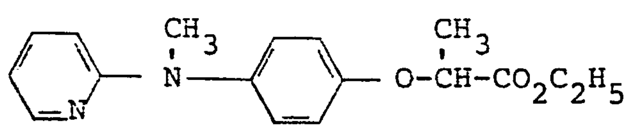

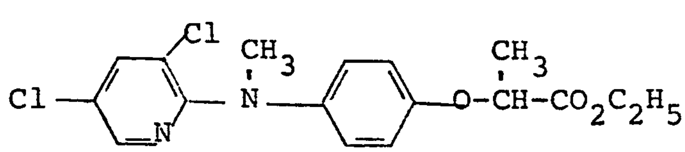

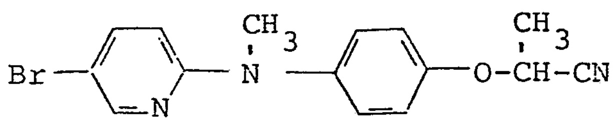

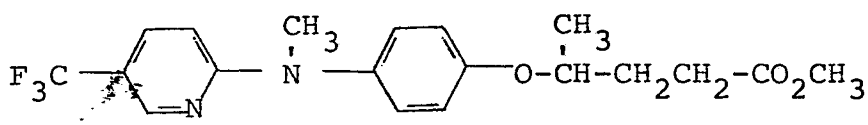

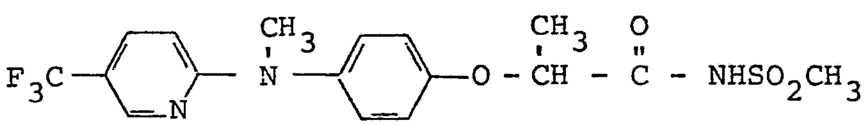

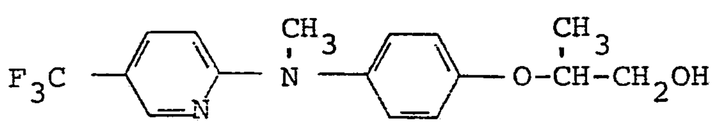

Specific examples of the compounds of the invention are detailed in Table 1 below.

TABLE 1

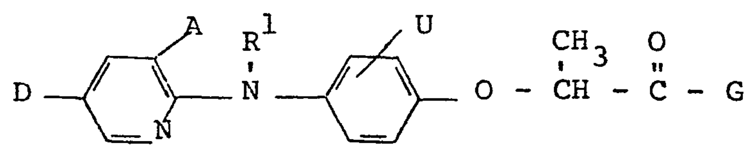

| Compound No | Substituents | | | | |
|---|---|---|---|---|---|
| | A | D | U | $R^1$ | G |
| 1 | Cl | Cl | H | $CH_3$ | $OCH_3$ |
| 2 | $NO_2$ | Cl | H | $CH_3$ | $OCH_3$ |
| 3 | Cl | $CF_3$ | H | $CH_3$ | $OCH_3$ |
| 4 | $NO_2$ | Cl | H | $C_2H_5$ | $OCH_3$ |
| 5 | H | $CF_3$ | H | $CH_3$ | $OCH_3$ |
| 6 | $NO_2$ | Br | H | $CH_3$ | $OCH_3$ |
| 7 | $NO_2$ | H | H | $CH_3$ | $OCH_3$ |
| 8 | H | $NO_2$ | H | $CH_3$ | $OCH_3$ |
| 9 | $NO_2$ | Cl | H | H | $OCH_3$ |
| 10 | $NO_2$ | H | H | H | $OCH_3$ |
| 11 | Br | $NO_2$ | H | H | $OCH_3$ |
| 12 | Cl | Cl | H | H | $OCH_3$ |
| 13 | H | Cl | H | $CH_3$ | $OCH_3$ |
| 14 | $NO_2$ | $CH_3$ | H | $CH_3$ | $OCH_3$ |
| 15 | Cl | Cl | 3-Cl | H | $OCH_3$ |
| 16 | H | Br | H | $CH_3$ | $OCH_3$ |
| 17 | $NO_2$ | Br | H | H | $OCH_3$ |
| 18 | Cl | Cl | 3-$NO_2$ | $CH_3$ | $OCH_3$ |
| 19 | Br | Br | H | $CH_3$ | $OCH_3$ |
| 20 | Cl | Cl | 3-Br | $CH_3$ | $OCH_3$ |
| 21 | $NH_2$ | Br | H | $CH_3$ | $OCH_3$ |
| 22 | H | H | H | $CH_3$ | $OCH_3$ |
| 23 | $NO_2$ | Br | H | $CH_3$ | $OC_4H_9$-n |

The compounds of the invention may be prepared by a variety of methods and in a further aspect the invention provides methods for the preparation of the compounds of formula I.

Compounds of formula Ia (I; $W=-\overset{O}{\overset{\|}{C}}-G$) wherein G is not hydroxy may be prepared from the acid of formula Ib($I; W=-CO_2H$) by any of the conventional methods known in the art for the conversion of a carboxylic acid to an acid salt, acid ester, acid amide or acid hydrazide (SCHEME A).

SCHEME A

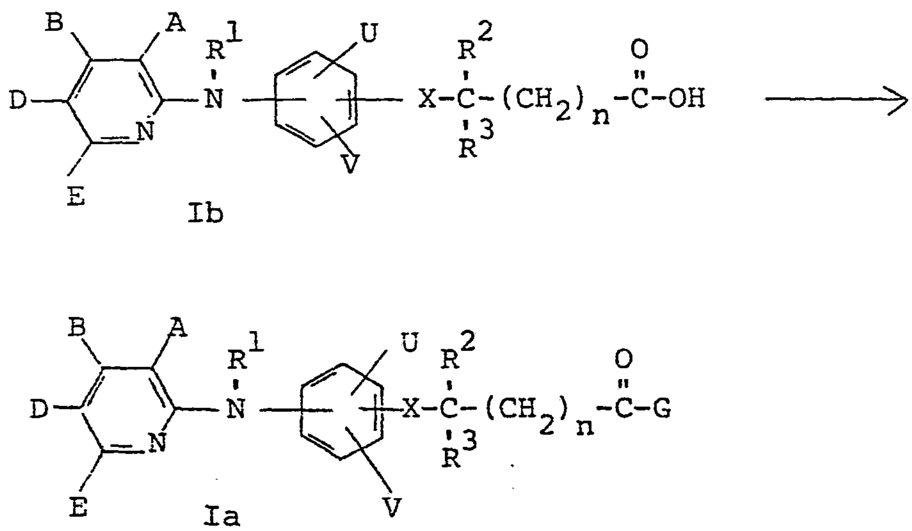

Ib

Ia

Compounds of formula Ic ($I; W=-C{\equiv}N$) may be prepared by any of the conventional methods known in the art for the conversion of an acid amide to the nitrile derivative of the acid (SCHEME B).

SCHEME B

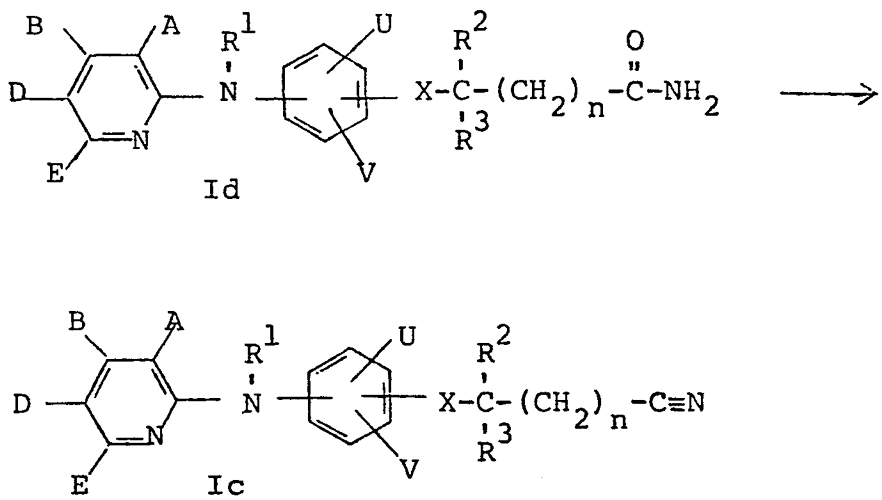

Id

Ic

Compounds of formula Ie (I; $W=-CH_2OH$) may be prepared from the acid or acid esters of formula If (I; $W=-\overset{\overset{O}{\|}}{C}-G$ wherein G = OH or O-alkyl) by any of the conventional methods known in the art for the conversion of an acid or acid ester to an alcohol (eg $LiAlH_4$ reduction). (SCHEME C).

SCHEME C

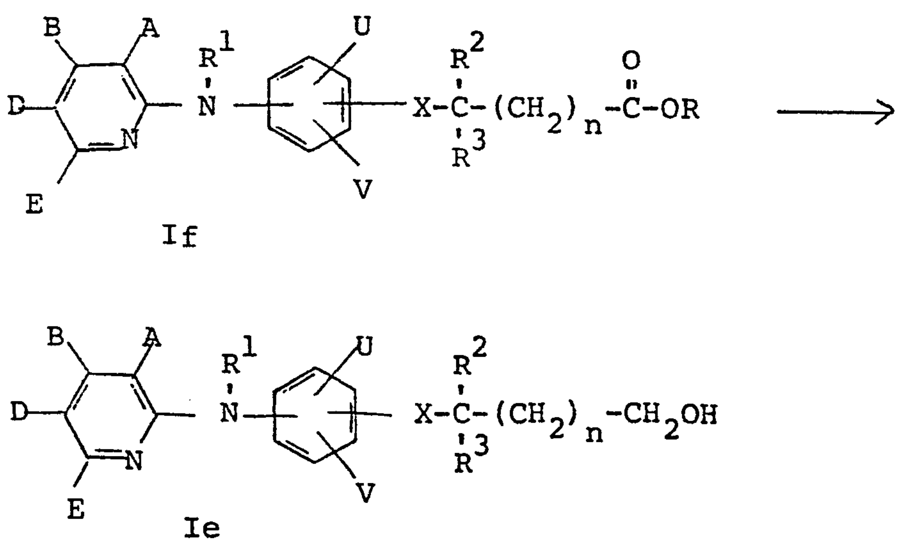

If

Ie

Alcohols of formula Ie (I; $W=-CH_2OH$) may be converted to alkyl halides (I; $W=-CH_2$-halogen) and ethers or thioethers (I; $W=-CH_2OR$ or $-CH_2SR$) by any of conventional methods known in the art.

Amines of formula Ig (I; $W=-CH_2NR^5R^6$) may be prepared either from the alkyl halides (I; $W=-CH_2$-halogen) or by reduction of the amides (I; $W=-\overset{O}{\overset{\|}{C}}-NR^5R^6$), both conventional processes known in the art.

Compounds of formula I wherein $R^1$ is not hydrogen may be prepared from compounds of formula I wherein $R^1$ is hydrogen by any of the conventional methods known in the art for the preparation of derivatives of secondary amines (eg alkylation and acylation).

Compounds of formula I wherein A, B, D, E, U, V, X, $R^1$, $R^2$, $R^3$, W and n are as hereinbefore defined may be prepared by the condensation of a phenol or thiophenol of formula IX with a compound of formula X wherein hal is chlorine, bromine or iodine, preferably in the presence of an alkaline material; according to SCHEME D.

SCHEME D

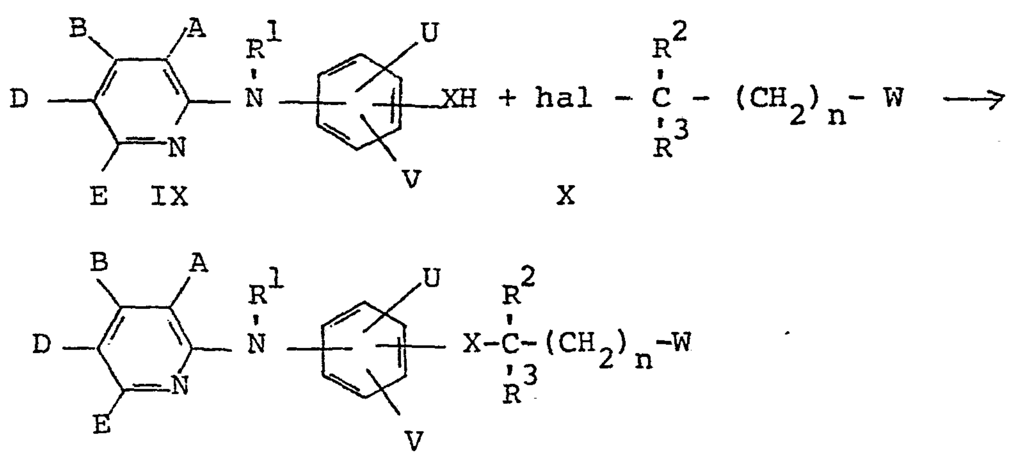

IX

X

I

Compounds of formula I may also be prepared by:

a) the condensation of the appropriate pyridine of formula V, wherein L is a leaving group (for example, alkylsulfonyl, chlorine, bromine or iodine) with the appropriate aniline of formula VI according to SCHEME E.

SCHEME E

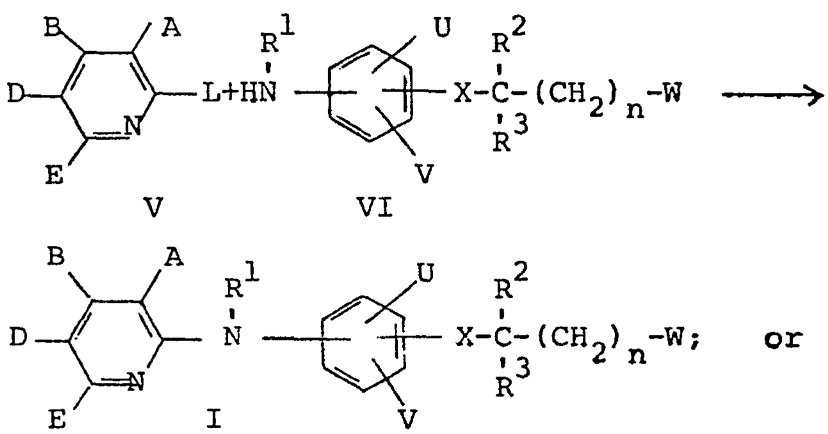

V VI

I

or

b) the following steps in sequence:

(i) the condensation of the appropriate pyridine of formula V, wherein L is a leaving group (for example, alkylsulfonyl, chlorine, bromine or iodine) with the appropriate aniline of formula VII, wherein Q is hydroxy, mercapto, $C_1$ to $C_6$ alkoxy or $C_1$ to $C_6$ alkylthio to give a compound of formula VIII wherein Q is hydroxy, mercapto, $C_1$ to $C_6$ alkoxy or $C_1$ to $C_6$ alkylthio;

(ii) the dealkylation of the compound of formula VIII prepared in step (i) above wherein Q is $C_1$ to $C_6$ alkoxy or $C_1$ to $C_6$ alkylthio to give a product of general formula IX; and

(iii) the condensation of product of formula IX

0013145

obtained in step (i) or step (ii) above with a compound of formula X according to the process described for SCHEME D above. (Steps (i) and (ii) are shown in SCHEME F).

SCHEME F

(i)

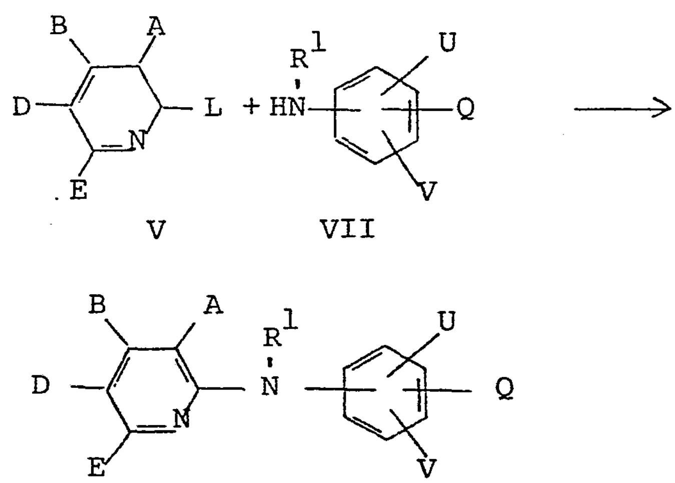

V VII

VIII

(ii)

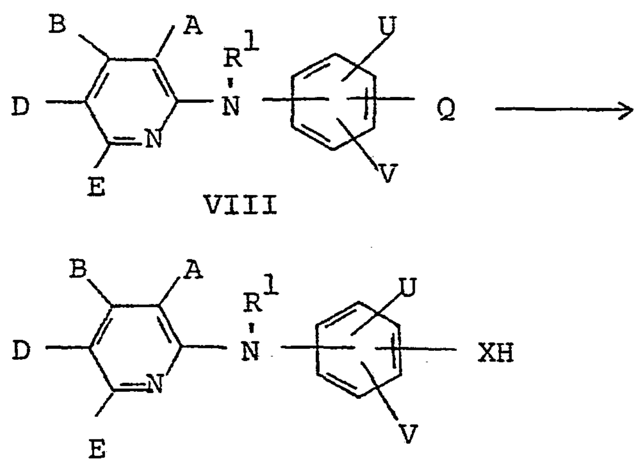

VIII

IX

The condensation reaction illustrated in SCHEME D and outlined above is preferably carried out in the presence of an alkaline material and preferably in the presence of a solvent. Suitable alkaline materials include, for example, the alkali and alkaline earth metal hydroxides and carbonates such as sodium hydroxide, potassium hydroxide, sodium carbonate and potassium carbonate. Suitable solvents include ketones such as, for example, acetone, methyl ethyl ketone and methyl isobutyl ketone, and dipolar aprotic solvents such as, for example, dimethylformamide, dimethylacetamide, dimethylsulfoxide, N-methylpyrrolidone, hexamethylphosphoramide and sulfolan.

The condensation reactions illustrated in SCHEMES E and F and outlined above are preferably carried out in the presence of a solvent.

The reaction conditions required to effect the condensation reactions illustrated in SCHEMES D, E and F and outlined above vary according to the nature of the reactants and the solvent used. In general the reaction is facilitated by the application of heat and usually a reaction temperature in the range of 40° to 150°C and reaction time of between 0.5 and 20 hours is satisfactory. However, higher or lower reaction temperatures and/or shorter or longer reaction times may be used if desired.

The dealkylation reaction illustrated in SCHEME F and outlined in paragraph b)(ii) above may be effected using a variety of reagents known in the art. For example, aryl-alkyl ethers may be cleaved using reagents such as pyridine hydrochloride, hydriodic acid, hydrobromic acid, sodium thioethoxide in dimethyl formamide, acetyl p-toluene-sulphonate, sodium or potassium iodide in formic or acetic acid, lithium iodide in 2,4,6-collidine and borontribromide. Reaction times and reaction conditions vary widely depending on the

dealkylation agent used and the ether to be cleaved. The reaction conditions generally employed when using the above "ether-cleavage" reagents are known to those skilled in the art and may be adapted without undue experimentation to effect the "ether-cleavage" reaction illustrated in SCHEME F and outlined in paragraph b)(ii) above.

The compounds of formula VIII

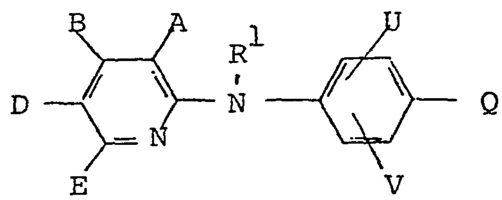

VIII

which are useful intermediates in the preparation of compounds of formula I, are novel compounds. Therefore, in a further embodiment the invention provides compounds of formula VII wherein A, B, D, E, $R^1$, U, V and Q are as hereinbefore defined.

The compounds of formula I are active as herbicides and therefore, in a further aspect the invention provides a process for severely damaging or killing unwanted plants which process comprises applying to the plants, or to the growth medium of the plants, an effective amount of a compound of formula I as hereinbefore defined.

Generally speaking the compounds of formula I are herbicidally effective against a variety of plants. However, certain of the compounds of the invention are selectively active against monocotyledonous plants, dicotyledonous plants being relatively unaffected by rates of application of the compounds of the invention which are severely damaging or lethal to other plant species. Therefore, in yet a further aspect the invention provides a process for selectively controlling the growth of weeds in crops which process comprises applying to the crop, or to the growth medium of the crop, a compound of formula I, as hereinbefore defined, in an amount insufficient to damage the crop substantially.

The compounds of formula I may be applied directly to the plant (post-emergence application) or to the soil before the emergence of the plant (pre-emergence application).

The compounds of formula I may be used on their own to inhibit the growth of, severely damage, or kill plants but are preferably used in the form of a composition comprising a compound of the invention in admixture with a carrier comprising a solid or liquid diluent. Therefore, in yet a further aspect the invention provides plant growth inhibiting, plant damaging, or plant killing compositions comprising a compound of formula I as hereinbefore defined and an inert carrier therefor.

Compositions according to the invention include both dilute compositions, which are ready for immediate use, and concentrated compositions, which require to be diluted before use, usually with water. Preferably the compositions contain from 0.01% to 90% by weight of the active ingredient. Dilute compositions ready for use preferably contain from 0.01 to 2% of active ingredient, while concentrated compositions may contain from 20 to 90% of active ingredient, although from 20 to 70% is usually preferred.

The solid compositions may be in the form of granules, or dusting powders wherein the active ingredient is mixed with a finely divided solid diluent, eg kaolin, bentonite, kieselguhr, dolomite, calcium carbonate, talc, powdered magnesia, Fuller's earth and gypsum. They may also be in the form of dispersible powders or grains, comprising a wetting agent to facilitate the dispersion of the powder or grains in liquid. Solid compositions in the form of a powder may be applied as foliar dusts.

Liquid compositions may comprise a solution or dispersion of an active ingredient in water optionally containing a surface-active agent, or may comprise a solution or dispersion of an active ingredient in a water-immiscible organic solvent which is dispersed as

droplets in water.

Surface-active agents may be of the cationic, anionic, or non-ionic type. The cationic agents are, for example, quaternary ammonium compounds (eg cetyltrimethylammonium bromide). Suitable anionic agents are soaps; salts of aliphatic mono esters of sulphuric acid, for example sodium lauryl sulphate; and salts of sulphonated aromatic compounds, for example sodium dodecylbenzenesulphonate, sodium, calcium, and ammonium lignosulphonate, butylnaphthalene sulphonate, and a mixture of the sodium salts of diisopropyl- and triisopropylnaphthalenesulphonic acid. Suitable non-ionic agents are the condensation products of ethylene oxide with fatty alcohols such as oleyl alcohol and cetyl alcohol, or with alkylphenols such as octyl- or nonylphenol or octylcresol. Other non-ionic agents are the partial esters derived from long chain fatty acids and hexitol anhydrides, for example sorbitan monolaurate; the condensation products of the partial ester with ethylene oxide; and the lecithins.

The aqueous solutions or dispersions may be prepared by dissolving the active ingredient in water or an organic solvent optionally containing wetting or dispersing agent(s) and then, when organic solvents are used, adding the mixtures so obtained to water optionally containing wetting or dispersing agent(s). Suitable organic solvents include, for example, ethylene dichloride, isopropyl alcohol, propylene glycol, diacetone alcohol, toluene, kerosene, methylnapthalene, the xylenes trichloroethylene.

The compositions for use in the form of aqueous solutions or dispersions are generally supplied in the form of a concentrate containing a high proportion of the active ingredient, and the concentrate is then diluted with water before use. These concentrates are usually

BAD ORIGINAL

required to withstand storage for prolonged periods and after such storage, to be capable of dilution with water to form aqueous preparations which remain homogeneous for a sufficient time to enable them to be applied by conventional spray equipment. Concentrates conveniently contain 20-90%, preferably 20-70%, by weight of the active ingredient(s). Dilute preparations ready for use may contain varying amounts of the active ingredient(s) depending upon the intended purpose; amounts of 0.01% to 10.0% and preferably 0.1% to 2%, by weight of active ingredient(s) are normally used.

A preferred form of concentrated composition comprises the active ingredient which has been finely divided and which has been dispersed in water in the presence of a surface-active agent and a suspending agent. Suitable suspending agents are hydrophilic colloids and include, for example, polyvinylpyrrolidone and sodium carboxymethylcellulose, and the vegetable gums, for example gum acacia and gum tragacanth. Preferred suspending agents are those which impart thixotropic properties to, and increase the viscosity of the concentrate. Examples of preferred suspending agents include hydrated colloidal mineral silicates, such as montmorillonite, beidellite, nontronite, hectorite, saponite, and saucorite. Bentonite is especially preferred. Other suspending agents include cellulose derivatives and polyvinyl alcohol.

The rate of application of the compounds of the invention will depend on a number of factors including, for example, the compound chosen for use, the identity of the plants whose growth is to be inhibited the formulation selected for use and whether the compound is to be applied for foliage or root uptake. As a general guide, however, an application rate of from 0.05 to 20 kilograms per hectare is suitable while from 0.1 to 10

kilograms per hectare may be preferred.

The compositions of the invention may comprise, in addition to one or more compounds of the invention, one or more compounds not of the invention but which possess biological activity. For example, as hereinbefore indicated the compounds of the invention are in general substantially more effective against monocotyledonous plants or grass species than against dicotyledonous plants or broad-leaved species. As a result, in certain applications the herbicidal use of the compounds of the invention alone may be insufficient to protect a crop. Accordingly in yet a still further embodiment the invention provides a herbicidal composition comprising a mixture of at least one herbicidal compound of formula I as hereinbefore defined with at least one other herbicide.

The other herbicide may be any herbicide not having the formula I. It will generally be a herbicide having a complementary action. For example, one preferred class is of mixtures comprising a herbicide active against broad-leaved weeds. A second preferred class is of mixtures comprising a contact herbicide.

Examples of useful complementary herbicides include:

A. benzo-2,1,3-thiadiazin-4-one-2,2-dioxides such as 3-isopropylbenzo-2,1,3-thiadiazin-4-one-2,2-dioxide (common name bentazon);

B. hormone herbicides and in particular the phenoxy - alkanoic acids such as 4-chloro-2-methylphenoxyacetic acid (common name MCPA), 2-(2,4-dichlorophenoxy)propionic acid (common name dichlorprop), 2,4,5-trichlorophenoxyacetic acid (common name 2,4,5-T), 4-(4-chloro-2-methylphenoxy)butyric acid (common name MCPB), 2,4-dichlorophenoxyacetic acid (common name 2,4-D), 4-(2,4-dichlorophenoxy)butyric

BAD ORIGINAL

acid (common name 2,4-DB), 2-(4-chloro-2-methylphenoxy)propionic acid (common name mecoprop), and their derivatives (eg salts, esters, amides and the like);

C. 3-[4-(4-halophenoxy)phenyl]-1,1-dialkylureas such as 3-[4-(4-chlorophenoxy)phenyl]-1,1-dimethylurea (common name chloroxuron);

D. dinitrophenols and their derivatives (eg acetates) such as 2-methyl-4,6-dinitrophenol (common name DNOC), 2-tertiarybutyl-4,6-dinitrophenol (common name dinoterb), 2-secondarybutyl-4,6-dinitrophenol (common name dinoseb) and its ester dinoseb acetate;

E. dinitroaniline herbicides such as N',N'-diethyl-2,6-dinitro-4-trifluoromethyl-*m*-phenylenediamine (common name dinitramine), 2,6-dinitro-N,N-dipropyl-4-trifluoromethylaniline (common name trifluralin) and 4-methylsulfonyl-2,6-dinitro-N,N-dipropylaniline (common name nitralin);

F. phenylurea herbicides such as N'-(3,4-dichlorophenyl)-N,N-dimethylurea (common name diuron) and N,N-dimethyl-N'-[3-(trifluoromethyl)phenyl]urea (common name fluometuron);

G. phenylcarbamoyloxyphenylcarbamates such as 3-[(methoxycarbonyl)amino]phenyl (3-methylphenyl)-carbamate (common name phenmedipham) and 3-[(ethoxycarbonyl)amino]phenyl phenylcarbamate (common name desmedipham);

H. 2-phenylpyridazin-3-ones such as 5-amino-4-chloro-2-phenylpyridazin-3-one (common name pyrazon);

I. uracil herbicides such as 3-cyclohexyl-5,6-trimethyleneuracil (common name lenacil), 5-bromo-3-*sec*-butyl-6-methyluracil (common name bromacil) and 3-*tert*-butyl-5-chloro-6-methyluracil (common

BAD ORIGINAL

name terbacil);

J. triazine herbicides such as 2-chloro-4-ethylamino-6-(iso-propylamino)-1,3,5-triazine (common name atrazine), 2-chloro-4,6-di(ethylamino)-1,3,5-triazine (common name simazine) and 2-azido-4-(iso-propylamino)-6-methylthio-1,3,5-triazine (common name aziprotryne);

K. 1-alkoxy-1-alkyl-3-phenylurea herbicides such as 3-(3,4-dichlorophenyl)-1-methoxy-1-methylurea (common name linuron), 3-(4-chlorophenyl)-1-methoxy-1-methylurea (common name monolinuron) and 3-(4-bromo-4-chlorophenyl)-1-methoxy-1-methylurea (common name chlorobromuron);

L. thiolcarbamate herbicides such as S-propyl dipropylthiocarbamate (common name vernolate);

M. 1,2,4-triazin-5-one herbicides such as 4-amino-4,5-dihydro-3-methyl-6-phenyl-1,2,4-triazine-5-one (common name metamitron) and 4-amino-6-tert-butyl-4,5-dihydro-3-methylthio-1,3,4-triazin-5-one (common name metribuzin);

N. benzoic acid herbicides such as 2,3,6-trichlorobenzoic acid (common name 2,3,6-TBA), 3,6-dichloro-2-methoxybenzoic acid (common name dicamba) and 3-amino-2,5-dichlorobenzoic acid (common name chloramben).

O. anilide herbicides such as N-butoxymethyl-α-chloro-2',6'-diethylacetanilide (common name butachlor)-the correspnding N-methoxy compound (common name alachlor), the corresponding N-iso-propyl compound (common name propachlor) and 3',4'-dichloropropionanilide (common name propanil);

P. dihalobenzonitrile herbicides such as 2,6-dichlorobenzonitrile (common name dichlobenil), 3,5-dibromo-4-hydroxybenzonitrile (common name bromoxynil) and 3,5-diiodo-4-hydroxybenzonitrile (common name ioxynil).

Q. haloalkanoic herbicides such as 2,2-dichloropropionic acid (common name dalapon), trichloroacetic acid (common name TCA) and salts thereof;

R. diphenylether herbicides such as 4-nitrophenyl 2-nitro-4-trifluoromethylphenyl ether (common name fluorodifen), methyl 5-(2,4-dichlorophenoxy)-2-nitrobenzoate (common name bifenox), 2-nitro-5-(2-chloro-4-trifluoromethylphenoxy)benzoic acid and 2-chloro-4-trifluoromethylphenyl 3-ethoxy-4-nitrophenyl ether; and

S. miscellaneous herbicides including N,N-dimethyldiphenylacetamide (common name diphenamid), N-(1-naphthyl)phthalamic acid (common name naptalam) and 3-amino-1,2,4-triazole.

Examples of useful contact herbicides include:

T. bipyridylium herbicides such as those in which the active entity is the 1,1'-dimethyl-4,4'-dipyridylium ion (common name paraquat) and those in which the active entity is the 1,1'-ethylene-2,2'-dipyridylium ion (common name diquat);

U. organoarsenical herbicides such as monosodium methanearsonate (common name MSMA); and

V. amino acid herbicides such as N-(phosphonomethyl)-glycine (common name glyphosate) and its salts and esters.

The invention is now illustrated by, but in no way limited to, the following Examples.

Example 1

Methyl 2-/4-(3,5-dichloro-N-methyl-2-pyridylamino) phenoxy/propionate (1)

(i) A mixture of 2-bromo-3,5-dichloropyridine (15.0 g) and p-anisidine (16.5 g) was heated at 160°C for 6 hours. The mixture was cooled and dissolved in a mixture of water and diethyl ether. The etherial layer was washed several times with dilute aqueous hydrochloric acid and was then dried over anhydrous magnesium sulfate. The ether was removed by distillation to give a light brown crystalline solid (16.4 g). Portion (4.0 g) of the brown solid was purified by column chromatography over silica gel (eluant diethyl ether) to give N-(4-methoxyphenyl)-3,5-dichloro-2-pyridylamine (3.9 g) as orange crystals (3.9 g), m.p. 70-74°C.

(ii) N-(4-Methoxyphenyl)-3,5-dichloro-2-pyridylamine (15.5 g) was dissolved in dimethylformamide and sodium hydride (4.3 g) was added. After the initial reaction had subsided methyl iodide (12.0 ml) was added and the mixture was stirred for 1 hour. The excess sodium hydride was destroyed by the careful addition of ethanol and then water was added to the mixture. The aqueous mixture was extracted with diethyl ether, the etherial extracts were dried over anhydrous magnesium sulfate and the ether was removed by distillation to give a brown oil. Portion of the oil (5.0 g) was purified by column chromatography over silica gel (eluant petroleum ether b.p. 40-60°C/diethyl ether). The product, a light brown oil (4.8 g), was characterised as N-(4-methoxyphenyl)-N-methyl-3,5-dichloro-2-pyridylamine by its p.m.r.

spectrum ($CDCl_3$; chemical shift δ ppm): 3.40, s(3H); 3.80, s(3H); 6.95, m(4H); 7.60, d(1H); 8.30, d(1H).

(iii) N-(4-Methoxyphenyl)-N-methyl-3,5-dichloro-2-pyridylamine (7.0 g) was dissolved in dichloromethane (90 ml) and the solution was cooled to a temperature of -78°C. Boron tribromide (8.0 ml) was slowly added to the mixture which was then allowed to come to room temperature and to stand at that temperature for a period of 1 hr. Water was then added and the mixture was extracted with diethyl ether. The etherial extract was dried and the ether was removed by distillation to give a yellow solid. The product was recrystallised from diethyl ether/petroleum ether (b.p. 40-60°C) to give 4-(3,5-dichloro-N-methyl-2-pyridylamino)-phenol (4.2 g), m.p. 138-140°C.

(iv) A mixture of 4-(3,5-dichloro-N-methyl-2-pyridylamino)phenol (2.0 g), methyl 2-bromopropionate (1.3 g), anhydrous potassium carbonate (1.13 g) and methyl ethyl ketone was heated under reflux for a period of 8 hr. Water was added and the mixture was extracted with diethyl ether. The etherial extract was dried over anhydrous magnesium sulfate and the ether was removed by distillation to give methyl 2-[4-(3,5-dichloro-N-methyl-2-pyridylamino)phenoxy]propionate (2.6 g) as a pale yellow oil. The product was characterised by its p.m.r. spectrum ($CDCl_3$; chemical shift δ ppm); 1.55, d(3H); 3.30, s(3H); 3.70, s(3H); 4.72, q(1H); 6.85, m(4H); 7.50, d(1H); 8.25 d(1H).

Example 2

The following compounds were prepared following essentially the same procedure as that described in Example 1.

a) Methyl 2-/4-(5-trifluoromethyl-N-methyl-2-pyridylamino)phenoxy/propionate (5) was prepared from 2-bromo-5-trifluoromethylpyridine, p-anisidine, methyl iodide and methyl 2-bromopropionate. The product, a pale yellow oil, was characterised by its p.m.r. spectrum ($CDCl_3$; chemical shift δ ppm): 1.63, d(3H); 3.43, s(3H); 3.76, s(3H); 4.83, q(1H); 6.40, d(1H); 6.8-7.7, m(5H); 8.45, br, s(1H).

b) Methyl 2-/4-(3-chloro-5-trifluoromethyl-N-methyl-2-pyridylamino)phenoxy/propionate (3) was prepared from 2-bromo-3-chloro-5-trifluoromethylpyridine, p-anisidine, methyl iodide and methyl 2-bromopropionate. The product, a pale yellow oil, was characterised by its p.m.r. spectrum ($CDCl_3$; chemical shift δ ppm): 1.60, d(3H); 3.35, s(3H); 3.76, s(3H); 4.80, q(1H), 6.96, m(4H); 7.70, d(1H); 8.53, d(1H).

c) Methyl 2-/4-(3,5-dichloro-2-pyridylamino)phenoxy/-propionate (12) was prepared from 2-bromo-3,5-dichloropyridine, p-anisidine and methyl 2-bromopropionate. The product was a solid, m.p. 98°C.

d) Methyl 2-/4-(5-chloro-N-methyl-2-pyridylamino)-phenoxy/propionate (13) was prepared from 2-bromo-5-chloropyridine, p-anisidine, methyl iodide and methyl 2-bromopropionate. The product, an oil, was characterised by its p.m.r. spectrum (chemical shift δ in ppm): 1.45, d(3H); 3.25, s(3H); 3.65, s(3H); 4.7, q(1H); 6.2, d(1H); 6.7-7.3, m(5H); 8.05, d(1H).

e) Methyl 2-/4-(5-bromo-N-methyl-2-pyridylamino)-phenoxy/propionate (16) was prepared from 2,5-dibromopyridine, p-anisidine, methyl iodide and methyl 2-bromopropionate. The product, an oil, was characterised by its p.m.r. spectrum (chemical shift δ in ppm): 1.6, d(3H); 3.3, s(3H); 3.8, s(3H); 4.8, q(1H); 6.3, d(1H); 6.7-7.5, m(5H); 8.2, d(1H).

f) Methyl 2-/4-(3,5-dibromo-N-methyl-2-pyridylamino)-phenoxy/propionate (19) was prepared from 2,3,5-tribromopyridine, p-anisidine, methyl iodide and methyl 2-bromopropionate. The product, an oil, was characterised by its p.m.r. spectrum (chemical shift δ in ppm): 1.6, d(3H); 3.3, s(3H); 3.8, s(3H); 4.8, q(1H); 6.8, m(4H); 7.9, d(1H); 8.3, d(1H).

g) Methyl 2-/4-(N-methyl-2-pyridylamino)phenoxy/-propionate (22) was prepared from 2-bromopyridine, p-anisidine, methyl iodide and methyl 2-bromopropionate. The product, an oil, was characterised by its p.m.r. spectrum (chemical shift δ in ppm): 1.6, d(3H); 3.4, s(3H); 3.7, s(3H); 4.8, q(1H); 6.45; m(2H); 7.05, m(5H); 8.25, d(1H).

Example 3

Methyl 2-/4-(5-chloro-3-nitro-N-methyl-2-pyridylamino)-phenoxy/propionate (2)

(i) A mixture of 2-bromo-5-chloro-3-nitropyridine (12.0 g), 4-(methylamino)phenol sulfate (18.2 g), water and acetonitrile were heated under reflux for a period of 3 days. The cooled aqueous mixture was then extracted with diethyl ether and the ethereal extract was dried over anhydrous magnesium sulfate. The solvent was removed by distillation to give a red solid which was purified by column chromatography over silica gel (eluant dichloromethane). The product (8.2 g), red crystals m.p. 154°C, was characterised as 4-(5-chloro-3-nitro-N-methyl-2-pyridylamino)phenol.

(ii) A mixture of 4-(5-chloro-3-nitro-N-methyl-2-pyridylamino)phenol (3.0 g), methyl 2-bromopropionate (1.8 g), anhydrous potassium carbonate (1.6 g) and methyl ethyl ketone was heated under reflux for a period of 4 hr. Water was added and the aqueous

mixture was extracted with diethyl ether. The etherial extract was dried over anhydrous magnesium sulfate and the ether was removed by distillation to give methyl 2-/4-(5-chloro-3-nitro-N-methyl-2-pyridylamino)phenoxy/propionate (3.9 g) as a red oil. The product was characterised by its p.m.r. spectrum ($CDCl_3$; chemical shift δ ppm): 1.60, d(3H); 3.53, s(3H); 3.76, s(3H); 4.76, q(1H); 6.93, m(4H); 8.00, d(1H); 8.43, d(1H).

Example 4

The following compounds were prepared using essentially the same procedure as that described in Example 3.

a) Methyl 2-/4-(5-bromo-3-nitro-N-methyl-2-pyridylamino)-phenoxy/propionate (6) was prepared from 2,5-dibromo-3-nitropyridine, 4-(methylamino)phenol sulfate and methyl 2-bromopropionate. The product was a red crystalline solid, m.p. 99°C.

b) Methyl 2-/4-(3-nitro-N-methyl-2-pyridylamino)phenoxy/ propionate (7) was prepared from 2-bromo-3-nitropyridine, 4-(methylamino)phenol sulfate and methyl 2-bromopropionate. The product, a red oil, was characterised by its p.m.r. spectrum ($CDCl_3$; chemical shift δ ppm): 1.60, d(3H); 3.50, s(3H); 3.70, s(3H); 4.70, q(1H); 6.83, m(5H); 7.86, br.d(1H); 8.40, br.d(1H).

c) Methyl 2-/4-(5-nitro-N-methyl-2-pyridylamino)-phenoxy/propionate (8) was prepared from 2-bromo-5-nitropyridine, 4-(methylamino)phenol sulfate and methyl 2-bromopropionate. The product was a yellow crystalline solid m.p. 91°C.

d) Methyl 2-/4-(5-methyl-3-nitro-N-methyl-2-pyridyl-amino)phenoxy/propionate (14) was prepared from 2-bromo-5-methyl-3-nitropyridine, 4-(methylamino)-

phenol sulfate and methyl 2-bromopropionate. The product, an oil, was characterised by its p.m.r. spectrum (chemical shift $\delta$ in ppm): 1.6, d(3H); 2.3, s(3H); 3.4, s(3H); 3.75, s(3H); 4.75, q(1H); 6.9, m(4H); 7.84, d(1H); 8.33, d(1H).

Example 5

Methyl 2-/4-(5-chloro-3-nitro-N-ethyl-2-pyridylamino)-phenoxy/propionate (4)

(i) A mixture of 2-bromo-5-chloro-3-nitropyridine (0.46 g), p-anisidine (0.36 g), concentrated hydrochloric acid (5 drops) water and methyl ethyl ketone was heated under reflux for a period of 24 hr. On cooling the product crystallised from the mixture and was collected by filtration and recrystallised from methyl ethyl ketone to give N-(4-methoxyphenyl)-5-chloro-3-nitro-2-pyridylamine (0.72 g) as reddish-black needles m.p. 120°C.

(ii) N-(4-Methoxyphenyl)-N-ethyl-5-chloro-3-nitro-2-pyridylamine was prepared from N-(4-methoxyphenyl)-5-chloro-3-nitro-2-pyridylamine and ethyl iodide using essentially the same procedure as that described in Example 1 part ii).

(iii) 4-(5-Chloro-3-nitro-N-ethyl-2-pyridylamino)phenol was prepared from N-(4-methoxyphenyl)-N-ethyl-5-chloro-3-nitro-2-pyridylamine using essentially the same procedure as that described in Example 1 part iii).

(iv) Methyl 2-/4-(5-chloro-3-nitro-N-ethyl-2-pyridylamino)phenoxy/propionate was prepared from 4-(5-chloro-3-nitro-N-ethyl-2-pyridylamino)phenol and methyl 2-bromopropionate using essentially the same procedure as that described in Example 1 part iv). The product, a red oil, was characterised by its p.m.r. spectrum ($CDCl_3$; chemical

shift δ ppm): 1.16, t(3H); 1.60, d(3H); 3.73, s(3H); 4.06, q(2H); 4.76, q(1H); 6.90, m(4H); 7.83, d(1H); 8.30, d(1H).

Example 6

Methyl 2-/4-(5-chloro-3-nitro-2-pyridylamino)phenoxy/-propionate (9)

i) A mixture of 2-bromo-5-chloro-3-nitropyridine (3.3 g), 4-aminophenol (4.7 g), concentrated sulfuric acid (4.2 g), water and acetonitrile was heated under reflux for a period of 2 days. The aqueous mixture was extracted with diethyl ether and the etherial extract was dried over anhydrous magnesium sulfate. The ether was removed by distillation and the residue, a red solid, was purified by column chromatography over silica gel (eluant dichloromethane). The product, 4-(5-chloro-3-nitro-2-pyridylamino)phenol was characterised by its p.m.r. spectrum ($CDCl_3$; chemical shift δ ppm): 6.86, d(1H); 7.40, d(1H); 8.3-8.9, m(4H): 9.30, br.s(1H); 10.00, br.s(1H).

ii) Methyl 2-/4-(5-chloro-3-nitro-2-pyridylamino)-phenoxy/propionate was prepared from 4-(5-chloro-3-nitro-2-pyridylamino)phenol and methyl 2-bromopropionate following essentially the same procedure as that described in Example 1 part iv). The product was a reddish-black crystalline solid, m.p. 118°C.

Example 7

The following compounds were prepared using essentially the same procedure as that described in Example 6.

a) Methyl 2-/4-(3-nitro-2-pyridylamino)phenoxy/-propionate (10) was prepared from 2-bromo-3-nitropyridine, 4-aminophenol and methyl 2-bromopropionate.

The product was a solid mp 100°C.

b) Methyl 2-/4-(3-bromo-5-nitro-2-pyridylamino)-phenoxy/propionate (11) was prepared from 2,3-dibromo-5-nitropyridine, 4-aminophenol and methyl 2-bromopropionate. The product was a solid, mp 118°C.

c) Methyl 2-/4-(5-bromo-3-nitro-2-pyridylamino)phenoxy/-propionate (17) was prepared from 2,5-dibromo-3-nitropyridine, 4-aminophenol and methyl 2-bromopropionate. The product, an oil, was characterised by its p.m.r. spectrum (chemical shift δ in ppm): 1.6, d(3H); 3.75, s(3H); 4.75, q(1H); 7.2, d of d(4H); 9.45, d(1H); 9.65, d(1H); 10.0, s(1H).

d) Methyl 2-/3-(5-bromo-3-nitro-2-pyridylamino)phenoxy/-propionate (24) was prepared from 2,5-dibromo-3-nitropyridine, 3-aminophenol and methyl 2-bromopropionate. The product was a solid, mp 120°C.

Example 8

Methyl 2-/3-chloro-4-(3,5-dichloro-2-pyridylamino)-phenoxy/propionate (15)

N-Chlorosuccinimide (0.86 g) was slowly added to a stirred solution of methyl 2-/4-(3,5-dichloro-2-pyridylamino)phenoxy/propionate (2.0 g, Example 2 c)) in dichloromethane. After stirring the mixture for a period of 18 hours at room temperature water was added and the organic layer was separated and dried (anhydrous $MgSO_4$). The solvent was evaporated under reduced pressure to give a brown solid which was recrystallised from ethanol to give methyl 2-/3-chloro-4-(3,5-dichloro-2-pyridylamino)phenoxy/propionate (1.15 g) as a colourless solid, mp 118°C.

Example 9

Methyl 2-/3-bromo-4-(3,5-dichloro-N-methyl-2-pyridylamino)phenoxy/propionate (20) was prepared from methyl 2-/4-(3,5-dichloro-N-methyl-2-pyridylamino)-phenoxy/propionate (Example 1) and N-bromosuccinimide

using essentially the same procedure as described in Example 8. The product, an oil, was characterised by its p.m.r. spectrum (chemical shift δ in ppm): 1.6, d(3H); 3.25, s(3H); 3.8, s(3H); 4.8, q(1H); 6.85, m(2H); m(2H); 7.2, d(1H); 7.5, d(1H); 8.2, d(1H).

Example 10

Methyl 2-/4-(3,5-dichloro-N-methyl-2-pyridylamino)-3-nitrophenoxy/propionate (18)

i) A mixture of N-(4-methoxyphenyl)-3,5-dichloro-2-pyridylamine (4.0 g; see Example 1 part i)), cupric nitrate trihydrate (1.8 g) and acetic acid (50 ml) was heated with stirring on a steam bath for a period of 2 hours. The mixture was poured into water and the aqueous solution was extracted with chloroform. The chloroform extracts were washed with water, dried (anhydrous $MgSO_4$) and the solvent was evaporated under reduced pressure to give a dark orange product. The crude product was purified by chromatography over silica gel with dichloromethane elution to give N-(4-methoxy-2-nitrophenyl)-3,5-dichloro-2-pyridylamine (3.9 g) as orange crystals, mp 158°C.

ii) Methyl 2-/4-(3,5-dichloro-N-methyl-2-pyridylamino)-3-nitrophenoxy/propionate was prepared from N-(4-methoxy-2-nitrophenyl)-3,5-dichloro-2-pyridylamine, methyl iodide and methyl 2-bromopropionate using essentially the same procedure as that described in Example 1, parts ii), iii) and iv). The product, an oil, was characterised by its p.m.r. spectrum (chemical shift δ in ppm): 1.65, d(3H); 3.45, s(3H); 3.85, s(3H); 4.9, q(1H); 7.2, d(2H); 7.5-7.7, m(2H); 8.1, d(1H).

Example 11

Methyl 2-/4-(3-amino-5-bromo-N-methyl-2-pyridylamino)-phenoxy/propionate (21)

i) A mixture of 4-(5-bromo-3-nitro-N-methyl-2-pyridylamino)phenol (3.0 g, Example 4 a)), stannous chloride (8.2 g), concentrated hydrochloric acid (30 ml) and ethanol (30 ml) was stirred at room temperature for a period of 2 days. The mixture was neutralised with aqueous sodium hydroxide and extracted with ethyl acetate. The extract was washed with water, dried (anhydrous $MgSO_4$) and the solvent evaporated under reduced pressure to give a brown solid. The crude solid was purified by column chromatography over silica gel with dichloromethane elution to give 4-(3-amino-5-bromo-N-methyl-2-pyridylamino)phenol (2.0 g) as pale brown crystals. The product was characterised by its p.m.r. spectrum (chemical shift δ in ppm): 3.2, s(3H); 4.4, m(2H); 6.65, m(4H); 6.9, d(1H); 7.55, d(1H); 8.80, s(1H).

ii) Methyl 2-/4-(3-amino-5-bromo-N-methyl-2-pyridylamino)phenoxy/propionate was prepared from 4-(3-amino-5-bromo-N-methyl-2-pyridylamino)phenol and methyl 2-bromopropionate using essentially the same procedure as that described in Example 1 part iv). The product, an oil, was characterised by its p.m.r. spectrum (chemical shift δ ppm): 1.6, d(3H); 3.2, s(3H); 3.7, s(3H); 3.8, br.s(2H); 4.7, q(1H); 6.7, m(4H); 6.9, d(1H); 7.8, d(1H).

Example 12

n-Butyl 2-/4-(5-bromo-3-nitro-N-methyl-2-pyridylamino)-phenoxy/propionate (23)

A mixture of methyl 2-/4-(5-bromo-3-nitro-N-methyl-2-pyridylamino)phenoxy/propionate (4.7 g, Example 4 a), concentrated sulfuric acid (5 drops) and n-butanol

(150 ml) was heated under reflux for a period of 18 hours. The excess n-butanol removed by distillation, dichloromethane was added to the residue and the mixture was washed several times with water. The dichloromethane solution was dried (anhydrous $MgSO_4$) and the solvent was evaporated to give n-butyl 2-[4-(5-bromo-3-nitro-N-methyl-2-pyridylamino)phenoxy]propionate as an oil (5.2 g) which was characterised by its p.m.r. spectrum (chemical shift δ in ppm): 0.9-1.6, m(7H); 1.6, d(3H); 3.5, s(3H); 4.2, t(2H); 4.75, q(1H); 6.95, m(4H); 8.1, d(1H); 8.5, d(1H).

Example 13

Concentrated formulations of the compounds of the invention were prepared by:

a) in the case of oils and waxy solids, dissolving the compound in toluene containing 7% v/v "Teric" N13 ("Teric" is a Trade Mark and "Teric" N13, a product of ethoxylation of nonylphenol, is available from ICI Australia Limited) and 3% v/v "Kemmat" SC15B ("Kemmat" is a Trade Mark and "Kemmat" SC15B is a formulation of calcium dodecylbenzene sulfonate); or

b) in the case of crystalline solids, adding 5 parts by weight of the compound and 1 part by weight of "Dyapol" PT ("Dyapol" is a Trade Mark and "Dyapol" PT is an anionic suspending agent) to 94 parts by weight of an aqueous solution containing 0.25% v/v of "Teric" N8 (a product of ethoxylation of nonylphenol) and ball-milling the mixture to produce a stable suspension.

The emulsifiable concentrates and suspensions were then diluted with water to give an aqueous composition of the required concentration suitable for use in the evaluation of the pre-emergence and post-emergence herbicidal activity of the compounds of the invention.

Example 14

The pre-emergent herbicidal activity of the compounds of the invention formulated as described in Example 13 was assessed by the following procedure.

The seeds of the test species were sown in rows 2 cm deep in soil contained in seed boxes. The monocotyledonous plants and the dicotyledonous plants were sown in separate boxes and after sowing the two boxes were sprayed with the required quantity of a composition of the invention. Two duplicate seed boxes were prepared in the same manner but were not sprayed with a composition of the invention and were used for comparison purposes. All the boxes were placed in a glasshouse, lightly watered with an overhead spray to initiate germination and then sub-irrigated as required for optimum plant growth. After three weeks the boxes were removed from the glasshouse and the effect of the treatment was visually assessed. The results are presented in Table 2 where the damage to plants is rated on a scale of from 0 to 3 where 0 represents from 0 to 25% damage, 3 represents 75 to 99% kill and 3+ represents 100% kill.

A dash (-) means that no experiment was carried out.

The names of the test plants are as follows:

| | |
|---|---|
| Wh | Wheat |
| Ot | Wild Oats |
| Rg | Ryegrass |
| Jm | Japanese millet |
| P | Peas |
| Ip | Ipomea |
| Ms | Mustard |
| Sf | Sunflower |

TABLE 2

PRE-EMERGENCE HERBICIDAL ACTIVITY

| Compound No | APPLICATION Rate (kg/ha) | TEST PLANT | | | | | | | |
|---|---|---|---|---|---|---|---|---|---|
| | | Wh | Ot | Rg | Jm | P | Ip | Ms | Sf |
| 1 | 5.0 | 0 | 0 | 3 | 2 | 0 | 0 | 0 | 0 |
| 1 | 1.0 | 0 | 0 | 2 | 0 | 0 | 0 | 0 | 0 |
| 2 | 5.0 | 0 | 2 | 3 | 3+ | 0 | 0 | 0 | 0 |
| 2 | 1.0 | 0 | 0 | 1 | 0 | 0 | 0 | 0 | 0 |
| 3 | 5.0 | 2 | 2 | 3 | 3+ | 0 | 0 | 0 | 0 |
| 3 | 1.0 | 0 | 1 | 2 | 2 | 0 | 0 | 0 | 0 |
| 4 | 5.0 | 0 | 0 | 0 | 2 | 0 | 0 | 0 | 0 |
| 5 | 5.0 | 0 | 0 | 3 | 3 | 0 | 0 | 0 | 0 |
| 6 | 5.0 | 3 | 3+ | 3+ | 3+ | 0 | 0 | 0 | 0 |
| 6 | 1.0 | 0 | 2 | 3 | 1 | 0 | 0 | 0 | 0 |
| 9 | 5.0 | 0 | 0 | 2 | 1 | 0 | 0 | 0 | 0 |
| 11 | 5.0 | 0 | 2 | 3 | 3 | 0 | 0 | 0 | 0 |
| 12 | 5.0 | 0 | 0 | 2 | 2 | 0 | 0 | 0 | 0 |
| 13 | 5.0 | 0 | 1 | 3 | 2 | 0 | 0 | 0 | 0 |
| 16 | 5.0 | 1 | 2 | 3+ | 3+ | 0 | 0 | 0 | 0 |
| 17 | 5.0 | 0 | 0 | 2 | 3 | 0 | 0 | 0 | 0 |
| 19 | 5.0 | 0 | 1 | 3 | 3+ | 0 | 0 | 0 | 0 |
| 23 | 5.0 | 0 | 2 | 3 | 3+ | 0 | 0 | 0 | 0 |

Example 15

The post-emergent herbicidal activity of the compounds of the invention formulated as described in Example 13 was assessed by the following procedure.

The seeds of the test species were sown in rows 2 cm deep in soil contained in seed boxes. The monocotyledonous plants and the dicotyledonous plants were sown in separate seed boxes in duplicate. The four seed boxes were placed in a glasshouse, lightly watered with an overhead spray to initiate germination and then sub-irrigated as required for optimum plant growth. After the plants had grown to a height of about 10 to 12.5 cm one box of each of the monocotyledonous plants and the dicotyledonous plants was removed from the glasshouse and sprayed with the required quantity of a composition of the invention. After spraying the boxes were returned to the glasshouse for further 3 weeks and the effects of treatment was visually assessed by comparison with the untreated controls. The results are presented in Table 3 where the damage to plants is rated on a scale of from 0 to 3 where 0 represents 0 to 25% damage, 3 represents 75 to 99% kill and 3+ represents 100% kill. A dash (-) means that no experiment was carried out.

The names of the test plants are as follows:

| | |
|---|---|
| Wh | Wheat |
| Ot | Wild Oats |
| Rg | Ryegrass |
| Jm | Japanese millet |
| P | Peas |
| Ip | Ipomea |
| Ms | Mustard |
| Sf | Sunflower |

TABLE 3

POST-EMERGENCE HERBICIDAL ACTIVITY

| Compound No | APPLICATION Rate (kg/ha) | TEST PLANT | | | | | | | |
|---|---|---|---|---|---|---|---|---|---|
| | | Wh | Ot | Rg | Jm | P | Ip | Ms | Sf |
| 1 | 5.0 | 2 | 3+ | 3 | 3+ | 0 | 1 | 2 | 2 |
| 1 | 1.0 | 2 | 1 | 3 | 3+ | 0 | 0 | 0 | 0 |
| 2 | 5.0 | 2 | 3 | 3 | 3+ | 0 | 0 | 0 | 0 |
| 2 | 1.0 | 1 | 2 | 2 | 3+ | 0 | 0 | 0 | 0 |
| 3 | 5.0 | 3+ | 3+ | 3+ | 3+ | 0 | 2 | 3+ | 2 |
| 3 | 1.0 | 2 | 3 | 3+ | 3+ | 0 | 0 | 0 | 0 |
| 4 | 5.0 | 3 | 3 | 3+ | 3+ | 0 | 0 | 0 | 0 |
| 4 | 1.0 | 0 | 0 | 1 | 1 | 0 | 0 | 0 | 0 |
| 5 | 5.0 | 3 | 1 | 3+ | 3+ | 0 | 0 | 0 | 0 |
| 5 | 1.0 | 0 | 0 | 3 | 3 | 0 | 0 | 0 | 0 |
| 5 | 0.5 | 0 | 0 | 3 | 3 | 0 | 0 | 0 | 0 |
| 6 | 5.0 | 3 | 3+ | 3+ | 3+ | 0 | 0 | 0 | 0 |
| 6 | 1.0 | 0 | 1 | 3 | 3+ | 0 | 0 | 0 | 0 |
| 9 | 5.0 | 0 | 2 | 3+ | 3+ | 0 | 0 | 0 | 0 |
| 12 | 5.0 | 2 | 2 | 3+ | 3+ | 0 | 0 | 0 | 0 |
| 12 | 1.0 | 1 | 0 | 3 | 3+ | 0 | 0 | 0 | 0 |
| 13 | 5.0 | 3 | 3+ | 3+ | 3+ | 0 | 0 | 3 | 2 |
| 13 | 1.0 | 2 | 3+ | 3+ | 3+ | 0 | 0 | 0 | 0 |
| 13 | 0.5 | 0 | - | 2 | 3 | 0 | 0 | 0 | 0 |
| 16 | 5.0 | 3 | - | 3+ | 3+ | 0 | 0 | 0 | 0 |
| 16 | 1.0 | 1 | - | 3 | 3+ | 0 | 0 | 0 | 0 |
| 16 | 0.5 | 0 | 2 | 3 | 3+ | 0 | 0 | 0 | 0 |
| 17 | 5.0 | 3 | - | 3+ | 3+ | 0 | 0 | 0 | 0 |
| 17 | 1.0 | 2 | - | 3 | 3+ | 0 | 0 | 0 | 0 |
| 17 | 0.5 | 0 | - | 3 | 3+ | 0 | 0 | 0 | 0 |

TABLE 3 Continued

| Compound No | APPLICATION Rate (kg/ha) | TEST PLANT | | | | | | | |
|---|---|---|---|---|---|---|---|---|---|
| | | Wh | Ot | Rg | Jm | P | Ip | Ms | Sf |
| 19 | 5.0 | 3 | 3 | 3+ | 3+ | 0 | 2 | 1 | 2 |
| 19 | 1.0 | 0 | 2 | 2 | 3 | 0 | 0 | 0 | 0 |
| 20 | 10.0 | 0 | 0 | 0 | 3 | 0 | 3+ | 3+ | 2 |
| 23 | 5.0 | 3+ | 3+ | 3+ | 3+ | 0 | 0 | 0 | 0 |
| 23 | 1.0 | 3 | 3 | 3 | 3 | 0 | 0 | 0 | 0 |

Example 16

The compounds were formulated for test by mixing an appropriate amount with 5 ml of an emulsion prepared by diluting 160 ml of a solution containing 21.8 g per litre of "Span" 80 and 78.2 g per litre of "Tween" 20 in methylcyclohexanone to 500 ml with water. "Span" 80 is a Trade Mark for a surface-active agent comprising sorbitan monolaurate. "Tween" 20 is a Trade Mark for a surface-active agent comprising a condensate of sorbitan monolaurate with 20 molar proportions of ethylene oxide. Each 5 ml emulsion containing a test compound was then diluted to 40 ml with water and sprayed on to young pot plants (post-emergence test) of the species named in Table 4 below. Damage to test plants was assessed after 14 days on a scale of 0 to 5 where 0 is 0 to 20% damage and 5 is complete kill. In a test for pre-emergence herbicidal activity, seeds of the test plants were sown in a shallow slit formed in the surface of soil in fibre trays. The surface was then levelled and sprayed, and fresh soil then spread thinly over the sprayed surface. Assessment of herbicidal damage was carried out after 21 days using the same scale of 0 to 5 as the post-emergence test. In both cases the degree of herbicidal damage was assessed by comparison with untreated control plants. The results are given in Table 4 below. A dash (-) means that no experiment was carried out.

The names of the test plants were as follows:

| | |
|---|---|
| Sb | Sugar beet |
| Rp | Rape |
| Ct | Cotton |
| Sy | Soy bean |
| Mz | Maize |
| Ww | Winter wheat |
| Rc | Rice |
| Sn | *Senecio vulgaris* |
| Ip | *Ipomea purpurea* |
| Am | *Amaranthus retroflexus* |
| Pi | *Polygonum aviculare* |
| Ca | *Chenopodium album* |
| Po | *Portulaca oleracea* |
| Xa | *Xanthium pensylvanicum* |
| Ab | *Abutilon theophrasti* |
| Cv | *Convolvulus arvensis* |
| Ot | Cultivated oats and wild oats (*Avena fatua*) Wild oats are used in the post-emergence test and cultivated oats in the pre-emergence test |
| Dg | *Digitaria sanguinalis* |
| Pu | *Poa annua* |
| St | *Setaria viridis* |
| Ec | *Echinochloa crus-galli* |
| Sh | *Sorghum halepense* |
| Ag | *Agropyron repens* |
| Cn | *Cyperus rotundus* |

TABLE 4 - PART A

| Compound No | APPLICATION Method | Rate (kg/ha) | Sb | Rp | Ct | Sy | Mz | Ww | Rc | Sn | Ip | Am | Pi | Ca |
|---|---|---|---|---|---|---|---|---|---|---|---|---|---|---|
| 1 | PRE | 5.0 | 0 | 2 | 0 | 0 | 3 | 2 | 4 | 3 | 2 | 1 | - | - |
| 1 | PRE | 0.8 | 1 | 0 | 0 | 0 | 0 | 0 | 0 | 1 | 1 | - | - | - |
| 1 | POST | 5.0 | 4 | 3 | 1 | 3 | 4 | 2 | 0 | 3 | 1 | 5 | - | 4 |
| 1 | POST | 0.8 | 3 | 3 | 2 | 3 | 3 | 1 | 0 | 2 | 0 | 3 | 1 | 1 |
| 2 | PRE | 5.0 | 2 | 0 | 0 | 1 | 2 | 2 | 4 | 0 | 0 | 0 | - | 0 |
| 2 | PRE | 1.0 | 0 | 0 | 0 | 0 | 2 | 0 | 2 | 0 | 0 | 0 | - | 0 |
| 2 | POST | 5.0 | 0 | 0 | 0 | 0 | 4 | 2 | 2 | 0 | 0 | 4 | 0 | 1 |
| 2 | POST | 1.0 | 0 | 0 | 0 | 0 | 4 | 1 | 0 | 0 | 0 | 1 | 0 | 2 |
| 3 | PRE | 5.0 | 1 | 3 | 0 | 0 | 3 | 3 | 5 | 2 | 0 | 4 | - | - |
| 3 | PRE | 1.0 | 0 | 0 | 0 | 0 | 2 | 1 | 3 | 0 | 0 | 2 | - | - |
| 3 | POST | 5.0 | 3 | 3 | 0 | 3 | 4 | 3 | 1 | 3 | 1 | 4 | - | 3 |
| 3 | POST | 1.0 | 3 | 2 | 0 | 2 | 3 | 2 | 0 | 2 | 0 | - | - | - |
| 5 | PRE | 5.0 | 1 | 1 | 0 | 0 | 4 | 4 | 3 | 2 | 0 | 4 | 0 | - |
| 5 | PRE | 1.0 | 1 | 1 | 0 | 0 | 5 | 3 | 2 | 1 | 0 | 0 | 0 | - |
| 5 | POST | 5.0 | 3 | 4 | 1 | 3 | 5 | 4 | 0 | 4 | 0 | 4 | 2 | 3 |
| 5 | POST | 1.0 | 2 | 3 | 0 | 2 | 5 | 2 | 0 | 3 | 0 | 3 | 0 | 2 |
| 6 | PRE | 5.0 | 1 | 0 | 0 | 0 | 3 | 3 | 5 | 0 | 0 | 0 | 0 | 0 |
| 6 | PRE | 1.0 | 1 | 0 | 0 | 0 | 2 | 2 | 4 | 1 | 0 | 0 | 0 | 0 |
| 6 | POST | 5.0 | 1 | 1 | 0 | 0 | 5 | 4 | 0 | 0 | 0 | 4 | 0 | 0 |
| 6 | POST | 1.0 | 0 | 0 | 0 | 0 | 5 | 2 | 0 | 0 | 0 | 1 | 0 | 0 |
| 13 | PRE | 5.0 | 0 | 0 | 0 | 0 | 4 | 3 | 3 | 0 | 0 | 0 | 0 | 0 |
| 13 | PRE | 1.0 | 0 | 0 | 0 | 0 | 3 | 0 | 1 | 0 | 0 | 0 | 0 | - |
| 14 | PRE | 5.0 | 0 | 0 | 0 | 0 | 1 | 0 | 1 | 4 | 0 | 0 | 2 | 0 |
| 14 | POST | 5.0 | 0 | - | 0 | 1 | 2 | 0 | 0 | 0 | 0 | 1 | - | 2 |

TABLE 4 - PART A Continued

| Compound No | APPLICATION Method | Rate (kg/ha) | TEST PLANT Sb | Rp | Ct | Sy | Mz | Ww | Rc | Sn | Ip | Am | Pi | Ca |
|---|---|---|---|---|---|---|---|---|---|---|---|---|---|---|
| 16 | PRE | 5.0 | 0 | 0 | 0 | 0 | 4 | 4 | 4 | 3 | 0 | 0 | 0 | 0 |
| 16 | PRE | 1.0 | 0 | 0 | 0 | 0 | 4 | 4 | 2 | 0 | 0 | 0 | 0 | 0 |
| 16 | POST | 5.0 | 2 | - | 4 | 4 | 5 | 4 | 2 | 5 | 0 | 5 | - | 4 |
| 16 | POST | 1.0 | 0 | - | 0 | 1 | 4 | 2 | 0 | 3 | 0 | 1 | - | 0 |
| 17 | PRE | 2.0 | 0 | 0 | 0 | 0 | 2 | 3 | 2 | 0 | 0 | 0 | 3 | - |
| 17 | PRE | 0.5 | - | 2 | 1 | 0 | 1 | 2 | 0 | - | 0 | 0 | 0 | - |
| 17 | POST | 2.0 | 0 | 2 | 1 | 0 | 5 | 4 | 3 | 0 | 5 | 0 | 0 | 5 |
| 17 | POST | 0.5 | 0 | 0 | 0 | 0 | 0 | 2 | 2 | - | 0 | 0 | 0 | 0 |
| 19 | PRE | 5.0 | 0 | 0 | 0 | 0 | 2 | 0 | 0 | 1 | 0 | 0 | 0 | 0 |
| 19 | PRE | 2.0 | 0 | 0 | 0 | 0 | 0 | 0 | 0 | 1 | 0 | 0 | 0 | 0 |
| 19 | POST | 5.0 | 3 | 4 | 1 | 4 | 5 | 3 | 0 | 4 | 0 | 4 | 2 | 2 |
| 19 | POST | 2.0 | 2 | 2 | 0 | 2 | 1 | 0 | 0 | 4 | 0 | 3 | 1 | 0 |

TABLE 4 - PART B

| Compound No | APPLICATION Method | Rate (kg/ha) | TEST PLANT Po | Xa | Ab | Cv | Ot | Dg | Pu | St | Ec | Sh | Ag | Cn |
|---|---|---|---|---|---|---|---|---|---|---|---|---|---|---|
| 1 | PRE | 5.0 | 3 | 0 | 0 | – | 0 | 3 | 0 | 3 | 3 | 3 | 0 | 0 |
| 1 | PRE | 0.8 | 2 | 2 | 2 | – | 0 | 0 | – | 0 | 0 | – | 0 | 2 |
| 1 | POST | 5.0 | 2 | 3 | 3 | 2 | 2 | 3 | 3 | 4 | 4 | 3 | 2 | 0 |
| 1 | POST | 0.8 | 0 | 1 | 1 | 0 | 0 | 1 | 0 | 3 | 3 | 4 | 1 | 0 |
| 2 | PRE | 5.0 | 0 | 0 | 0 | – | 2 | 4 | 0 | 5 | 3 | 3 | 5 | 0 |
| 2 | PRE | 1.0 | 0 | 0 | 0 | – | 0 | 1 | 0 | 2 | 0 | 2 | 1 | 0 |
| 2 | POST | 5.0 | 0 | 0 | 1 | 0 | 4 | 2 | 0 | 4 | 4 | – | 0 | 0 |
| 2 | POST | 1.0 | 0 | 0 | 0 | 0 | 0 | 0 | 0 | 3 | 1 | – | 0 | 0 |
| 3 | PRE | 5.0 | 3 | 0 | – | – | 3 | 4 | 0 | 4 | 4 | 5 | 4 | 0 |
| 3 | PRE | 1.0 | 0 | 0 | 1 | – | 0 | 3 | 0 | 4 | 2 | 3 | 0 | 0 |
| 3 | POST | 5.0 | 3 | 2 | 2 | 2 | 4 | 3 | 3 | 4 | 4 | 4 | 2 | 0 |
| 3 | POST | 1.0 | 2 | 1 | 1 | 1 | 5 | 1 | 1 | 4 | 3 | 4 | 1 | 0 |
| 5 | PRE | 5.0 | 1 | 0 | 1 | – | 4 | 4 | 3 | 4 | 4 | 4 | 3 | 0 |
| 5 | PRE | 1.0 | 0 | 0 | 0 | – | 3 | 4 | 2 | 4 | 4 | 3 | 0 | 0 |
| 5 | POST | 5.0 | 3 | 2 | 2 | 0 | 4 | 3 | 2 | 1 | 5 | 0 | 3 | 0 |
| 5 | POST | 1.0 | 2 | 0 | 0 | 0 | 4 | 0 | 0 | 0 | 3 | 0 | 1 | 0 |
| 6 | PRE | 5.0 | 0 | 0 | 0 | – | 3 | 4 | 3 | 4 | 4 | 4 | 5 | 0 |
| 6 | PRE | 1.0 | 0 | 0 | 0 | – | 1 | 3 | 0 | 3 | 0 | 3 | 0 | 0 |
| 6 | POST | 5.0 | 0 | 0 | 0 | 0 | 4 | 4 | 1 | 4 | 4 | 4 | 3 | 0 |
| 6 | POST | 1.0 | 0 | 0 | 0 | 0 | 3 | 4 | 0 | 3 | 3 | 2 | 2 | 0 |
| 13 | PRE | 5.0 | 1 | 0 | 0 | – | 3 | 4 | 0 | 4 | 4 | 3 | 2 | 0 |
| 13 | PRE | 1.0 | 0 | 0 | 0 | – | 3 | 4 | 0 | 2 | 3 | 0 | – | 0 |
| 14 | PRE | 5.0 | 0 | 0 | 0 | – | 5 | 2 | 4 | 1 | 1 | 1 | 0 | 0 |
| 14 | POST | 5.0 | 2 | 0 | 0 | 0 | 0 | 1 | 0 | 1 | 2 | 0 | 0 | 0 |

TABLE 4 - PART B Continued

| Compound No | APPLICATION Method | Rate (kg/ha) | Po | Xa | Ab | Cv | Ot | Dg | Pu | St | Ec | Sh | Ag | Cn |
|---|---|---|---|---|---|---|---|---|---|---|---|---|---|---|
| 16 | PRE | 5.0 | 0 | 0 | 0 | – | 4 | 4 | 2 | 4 | 4 | 3 | – | 0 |
| 16 | PRE | 1.0 | 0 | 0 | 0 | – | 4 | 4 | 0 | 4 | 4 | 2 | 0 | 0 |
| 16 | POST | 5.0 | 3 | 2 | 2 | 0 | 4 | 1 | 0 | 4 | 5 | – | 4 | 0 |
| 16 | POST | 1.0 | 0 | 0 | 1 | 2 | 0 | 0 | 0 | 1 | 4 | 0 | 1 | 0 |
| 17 | PRE | 2.0 | 1 | 0 | 0 | – | 4 | 4 | 2 | 4 | 3 | 2 | 3 | 0 |
| 17 | PRE | 0.5 | 0 | 0 | 0 | – | 2 | 0 | 0 | 1 | 0 | 2 | 1 | 0 |
| 17 | POST | 2.0 | 1 | 0 | 0 | – | 4 | 5 | 1 | 4 | 5 | 5 | 2 | 0 |
| 17 | POST | 0.5 | 1 | 0 | 0 | 0 | 3 | 3 | 0 | 2 | 3 | 4 | 1 | 0 |
| 19 | PRE | 5.0 | 0 | 0 | 0 | – | 0 | 1 | 0 | 1 | 4 | 0 | 0 | 0 |
| 19 | PRE | 2.0 | 0 | 0 | – | – | 0 | 0 | 0 | 0 | 0 | 0 | 0 | 0 |
| 19 | POST | 5.0 | 2 | 2 | 3 | 2 | 1 | 0 | 0 | 5 | 4 | 2 | 2 | 0 |
| 19 | POST | 2.0 | 1 | 0 | 0 | 0 | 0 | 0 | 0 | 5 | 2 | 0 | 1 | 0 |

1. A compound of formula I

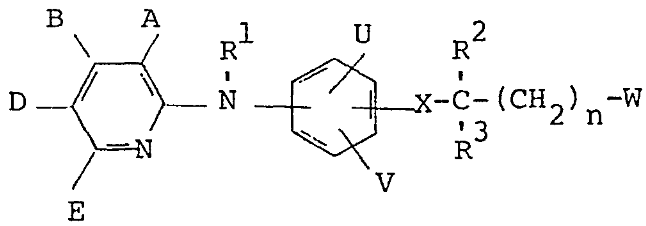

I

or a salt thereof wherein:

A, B, D, E, U and V are independently chosen from the group consisting of hydrogen, halogen, nitro, cyano, thiocyano, amino, $C_1$ to $C_6$ alkylamino, di($C_1$ to $C_6$ alkyl)-amino, $C_1$ to $C_6$ alkyl, $C_1$ to $C_6$ haloalkyl, $C_2$ to $C_6$ alkenyl, $C_3$ to $C_7$ cycloalkyl, $C_1$ to $C_6$ alkoxy, $C_1$ to $C_6$ haloalkoxy, $C_1$ to $C_6$ alkylthio, $C_1$ to $C_6$ alkylsulfinyl, $C_1$ to $C_6$ alkylsulfonyl, $C_1$ to $C_6$ haloalkylsulfinyl, $C_1$ to $C_6$ haloalkylsulfonyl, sulfo, $C_1$ to $C_6$ alkoxysulfonyl, sulfamoyl, N-($C_1$ to $C_6$ alkyl)sulfamoyl, N,N-di($C_1$ to $C_6$ alkyl)sulfamoyl, carboxy, ($C_1$ to $C_6$ alkoxy)carbonyl, carbamoyl, N-($C_1$ to $C_6$ alkyl)carbamoyl, N,N-di($C_1$ to $C_6$ alkyl)carbamoyl, phenyl, phenoxy, phenylthio, and the groups substituted phenyl, substituted phenoxy and substituted phenylthio wherein in each group the phenyl ring is substituted with one to three substituents chosen from the group consisting of halogen, $C_1$ to $C_6$ alkyl, $C_1$ to $C_6$ haloalkyl, $C_1$ to $C_6$ alkoxy, nitro and cyano;

$R^1$ is chosen from the group consisting of hydrogen, $C_1$ to $C_{10}$ alkyl, $C_2$ to $C_{10}$ alkenyl, $C_2$ to $C_{10}$ alkynyl, $C_2$ to $C_{10}$ alkoxyalkyl, $C_1$ to $C_{10}$ haloalkyl, formyl, $C_2$ to $C_{10}$ alkanoyl, phenyl, benzyl, benzoyl, and the groups phenyl, benzyl and benzoyl wherein in each group the phenyl ring is substituted with one or two substituents chosen from the group consisting of halogen, $C_1$ to $C_6$ alkyl, $C_1$ to $C_6$ haloalkyl, $C_1$ to $C_6$ alkoxy, nitro and cyano;

$R^2$ is chosen from the group consisting of hydrogen,

$C_1$ to $C_6$ alkyl, $C_2$ to $C_6$ alkenyl, $C_2$ to $C_6$ alkoxyalkyl, $C_1$ to $C_6$ haloalkyl, acetyl, propionyl, and $C_2$ to $C_6$ alkoxycarbonyl;

$R^3$ is chosen from the group consisting of hydrogen, $C_1$ to $C_6$ alkyl, $C_2$ to $C_6$ alkenyl, $C_2$ to $C_6$ alkoxyalkyl and $C_1$ to $C_6$ haloalkyl, or $R^2$ and $R^3$ together may form a methylene, ethylidene, propylidene or isopropylidene group;

W is chosen from the group consisting of cyano, thiocarbamoyl, $-\overset{O}{\overset{\|}{C}}-G$ and $CH_2Z$ wherein:

G is chosen from the group consisting of hydroxy, mercapto, $C_1$ to $C_{10}$ alkoxy, $C_1$ to $C_{10}$ haloalkoxy, $C_2$ to $C_{10}$ alkenyloxy, $C_2$ to $C_{10}$ alkynyloxy, $C_1$ to $C_{10}$ alkylthio, $C_2$ to $C_{10}$ alkenylthio, $C_2$ to $C_{10}$ alkynylthio, $C_3$ to $C_7$ cycloalkoxy, $C_3$ to $C_7$ cycloalkoxy substituted with one or two $C_1$ to $C_4$ alkyl groups, phenoxy, phenylthio, benzyloxy, benzylthio, the group $C_1$ to $C_{10}$ alkoxy substituted with a substituent chosen from the group consisting of $C_1$ to $C_6$ alkoxy, amino, ammonio, cyano, N-($C_1$ to $C_6$ alkyl)amino, N,N-di($C_1$ to $C_6$ alkyl)amino, and N,N,N-tri($C_1$ to $C_6$ alkyl)ammonio, the groups phenoxy, phenylthio, benzyloxy and benzylthio wherein in each group the phenyl ring is substituted with one or two substituents chosen from the group consisting of halogen, nitro, cyano, $C_1$ to $C_6$ alkyl, $C_1$ to $C_6$ haloalkyl and $C_1$ to $C_6$ alkoxy, the group OM wherein M is the cation of an inorganic or organic base, the group $-NHSO_2R^4$ wherein $R^4$ is chosen from $C_1$ to $C_{10}$ alkyl and $C_1$ to $C_{10}$ haloalkyl, and the group $-NR^5R^6$ wherein $R^5$ and $R^6$ are independently chosen from the group consisting of hydrogen, $C_1$ to $C_6$ alkyl, phenyl and benzyl or $R^5$ and $R^6$ together form a heterocyclic ring; and Z is chosen from halogen, hydroxy, mercapto, $C_1$ to $C_{10}$ alkoxy, $C_1$ to $C_{10}$ haloalkoxy, $C_1$ to $C_{10}$ alkylthio and the group $NR^5R^6$ wherein $R^5$ and $R^6$ are as hereinbefore de-

fined;

X is chosen from oxygen and sulfur; and

n is 0, 1 or 2.

2. A compound according to claim 1 wherein:

A, B, D, E, U and V are independently chosen from the group consisting of hydrogen, halogen, nitro, cyano, thiocyano, amino, $C_1$ to $C_6$ alkylamino, di($C_1$ to $C_6$ alkyl)amino, $C_1$ to $C_6$ alkyl, $C_1$ to $C_6$ haloalkyl, $C_2$ to $C_6$ alkenyl, $C_3$ to $C_7$ cycloalkyl, $C_1$ to $C_6$ alkoxy, $C_1$ to $C_6$ alkylthio, ($C_1$ to $C_6$ alkoxy)carbonyl, phenyl, phenoxy, phenylthio, and the groups substituted phenyl, substituted phenoxy and substituted phenylthio wherein in each group the phenyl ring is substituted with one to three substituents chosen from the group consisting of halogen, $C_1$ to $C_6$ alkyl, $C_1$ to $C_6$ haloalkyl, $C_1$ to $C_6$ alkoxy, nitro and cyano;

$R^1$ is chosen from the group consisting of hydrogen, $C_1$ to $C_{10}$ alkyl, $C_2$ to $C_{10}$ alkenyl, $C_2$ to $C_{10}$ alkoxyalkyl, $C_1$ to $C_{10}$ haloalkyl, formyl, $C_2$ to $C_{10}$ alkanoyl, phenyl, benzyl, benzoyl, and the groups phenyl, benzyl and benzoyl wherein in each group the phenyl ring is substituted with one or two substituents chosen from the group consisting of halogen, $C_1$ to $C_6$ alkyl, $C_1$ to $C_6$ haloalkyl, $C_1$ to $C_6$ alkoxy, nitro and cyano;

$R^2$ is chosen from the group consisting of hydrogen, $C_1$ to $C_6$ alkyl, $C_2$ to $C_6$ alkenyl, $C_2$ to $C_6$ alkoxyalkyl, $C_1$ to $C_6$ haloalkyl, acetyl, propionyl and $C_2$ to $C_6$ alkoxy carbonyl;

$R^3$ is chosen from the group consisting of hydrogen, $C_1$ to $C_6$ alkyl, $C_2$ to $C_6$ alkenyl, $C_2$ to $C_6$ alkoxyalkyl, and $C_1$ to $C_6$ haloalkyl or $R^2$ and $R^3$ together form a methylene, ethylidene, propylidene or isopropylidene group;

W is chosen from the group consisting of cyano, thiocarbamoyl, $-\overset{O}{\overset{\|}{C}}-G$ and $-CH_2Z$ wherein:

G is chosen from the group consisting of hydroxy, mercapto, $C_1$ to $C_{10}$ alkoxy, $C_1$ to $C_{10}$ haloalkoxy, $C_2$ to $C_{10}$ alkenyloxy, $C_2$ to $C_{10}$ alkynyloxy, $C_1$ to $C_{10}$ alkylthio, $C_2$ to $C_{10}$ alkenylthio, $C_3$ to $C_7$ cycloalkoxy, $C_3$ to $C_7$ cycloalkoxy substituted with one or two $C_1$ to $C_4$ alkyl groups, phenoxy, phenylthio, benzyloxy, benzylthio, the group $C_1$ to $C_{10}$ alkoxy substituted with a $C_1$ to $C_6$ alkoxy group, the groups phenoxy, phenylthio, benzyloxy and benzylthio wherein in each group the phenyl ring is substituted with one or two substituents chosen from the group consisting of halogen, nitro, cyano, $C_1$ to $C_6$ alkyl, $C_1$ to $C_6$ haloalkyl and $C_1$ to $C_6$ alkoxy, the group OM wherein M is the cation of an inorganic or organic base, the group $-NHSO_2R^4$ wherein $R^4$ is chosen from $C_1$ to $C_{10}$ alkyl and $C_1$ to $C_{10}$ haloalkyl, and the group $-NR^5R^6$ wherein $R^5$ and $R^6$ are independently chosen from the group consisting of hydrogen, $C_1$ to $C_6$ alkyl, phenyl and benzyl or $R^5$ and $R^6$ together form a heterocyclic ring; Z is chosen from the group consisting of halogen, hydroxy, mercapto, $C_1$ to $C_{10}$alkoxy, $C_1$ to $C_{10}$ haloalkoxy, $C_1$ to $C_{10}$ alkylthio and the group $NR^5R^6$ wherein $R^5$ and $R^6$ are independently chosen from the group consisting of hydrogen, $C_1$ to $C_6$ alkyl, phenyl and benzyl or $R^5$ and $R^6$ together form a heterocyclic ring;

X is chosen from oxygen and sulfur; and

n is 0, 1 or 2.

3. A compound according to claim 1 wherein:

A, B, D and E are independently chosen from the group consisting of hydrogen, halogen, nitro, cyano, amino, $C_1$ to $C_6$ alkyl and $C_1$ to $C_6$ haloalkyl;

U and V are independently chosen from hydrogen, halogen

and nitro;

$R^1$ is chosen from the group consisting of hydrogen, $C_1$ to $C_6$ alkyl, $C_2$ to $C_6$ alkenyl, $C_2$ to $C_6$ alkynyl, $C_2$ to $C_6$ alkanoyl and benzyl;

$R^2$ and $R^3$ are independently chosen from hydrogen and $C_1$ to $C_6$ alkyl;

W is chosen from the groups $-\overset{O}{\overset{\|}{C}}-G$ and $-CH_2Z$ wherein: G is chosen from the group consisting of hydroxy, $C_1$ to $C_{10}$ alkoxy, $C_2$ to $C_{10}$ alkenyloxy, $C_2$ to $C_{10}$ alkynyloxy, $C_3$ to $C_7$ cycloalkoxy, benzyloxy, $C_1$ to $C_{10}$ alkoxy substituted with a substituent chosen from amino, ammonio, N-($C_1$ to $C_6$ alkyl)amino, N,N-di($C_1$ to $C_6$ alkyl)amino and N,N,N-tri($C_1$ to $C_6$ alkyl)ammonio, and the group OM wherein M is an alkali metal or alkaline earth metal ion; Z is chosen from the group consisting of halogen, hydroxy, mercapto and $C_1$ to $C_6$ alkoxy;

X is oxygen; and

n is 0 or 2.

4. A compound according to any one of claims 1 to 3 inclusive of formula II

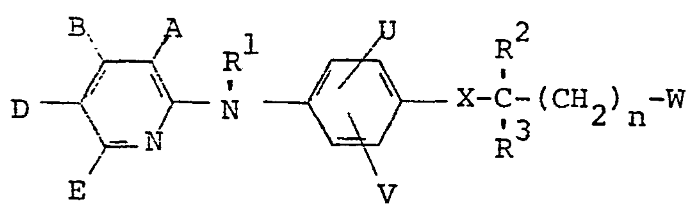

II.

5. A compound according to any one of claims 1, 3 or 4 wherein:

A is chosen from the group consisting of hydrogen, halogen, nitro and $C_1$ to $C_6$ haloalkyl;

D is chosen from the group consisting of hydrogen, halogen,

nitro, $C_1$ to $C_6$ alkyl and $C_1$ to $C_6$ haloalkyl;

$R^1$ is chosen from the group consistingof hydrogen, $C_1$ to $C_6$ alkyl, $C_2$ to $C_6$ alkenyl, $C_2$ to $C_6$ alkynyl and benzyl;

U is chosen from the group consisting of hydrogen, halogen and nitro;

$R^2$ is chosen from hydrogen and $C_1$ to $C_6$ alkyl;

B, E, V and $R^3$ are hydrogen;

W is the group $-\overset{O}{\overset{\|}{C}}-G$ wherein G is chosen from the group consisting of hydroxy, $C_1$ to $C_6$ alkoxy, $C_2$ to $C_6$ alkenyloxy, $C_2$ to $C_6$ alkynyloxy and $C_1$ to $C_6$ alkoxy substituted with a group chosen from N,N-di($C_1$ to $C_6$ alkyl)amino and N,N,N-tri($C_1$ to $C_6$ alkyl)ammonio;

X is oxygen; and

n is 0.

6. A compound according to any one of claims 1 to 5 inclusive wherein:

A is chosen from the group consisting of hydrogen, chlorine, bromine and nitro;

D is chosen from the group consisting of chlorine, bromine, nitro, methyl and trifluoromethyl;

$R^1$ is chosen from hydrogen, methyl and ethyl;

$R^2$ is methyl;

B, E, U, V and $R^3$ are hydrogen;

W is the group $-\overset{O}{\overset{\|}{C}}-G$ wherein G is chosen from $C_1$ to $C_6$ alkoxy;

X is oxygen; and

n is 0.

7. A compound according to any one of claims 1 to 5 inclusive wherein:

A is chosen from the group consisting of hydrogen, chlorine, bromine and nitro;

D is chosen from the group consistingof chlorine, bromine and trifluoromethyl;

$R^1$ is chosen from the group consisting of hydrogen and methyl;

$R^2$ is methyl;

B, E, U, V and $R^3$ are hydrogen;

W is the group $-\overset{O}{\overset{\|}{C}}-G$ wherein G is chosen from $C_1$ to $C_6$ alkoxy;

X is oxygen; and

n is 0.

8. A compound according to any one of claims 1 to 7 inclusive chosen from the group consisting of:

methyl 2-/4-(3-chloro-5-trifluoromethyl-N-methyl-2-pyridylamino)phenoxy7propionate;

methyl 2-/4-(5-trifluoromethyl-N-methyl-2-pyridylamino)-phenoxy7propionate;

methyl 2-/4-(5-bromo-N-methyl-3-nitro-2-pyridylamino)-phenoxy7propionate;

methyl 2-/4-(5-chloro-N-methyl-2-pyridylamino)phenoxy7-propionate;

methyl 2-/4-(5-bromo-N-methyl-2-pyridylamino)phenoxy7-propionate; and

methyl 2-/4-(5-bromo-3-nitro-2-pyridylamino)phenoxy7-propionate.

9. A compound of formula VIII

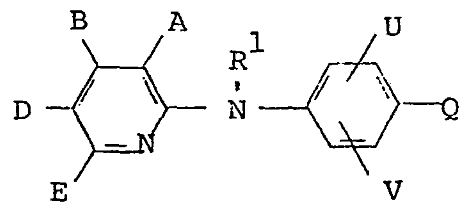

VIII

wherein A, B, D, E, U, V and $R^1$ are as defined according to any one of claims 1 to 7 inclusive and Q is chosen from hydroxy, mercapto, $C_1$ to $C_6$ alkoxy and $C_1$ to $C_6$ alkylthio.

10. A herbicidal composition comprising as active ingredient a compound as defined according to any one of claims 1 to 8 inclusive and a carrier therefor.

11. A composition according to claim 10 wherein said composition is in the form of a liquid and comprises a surface active agent.

12. A composition according to claim 10 wherein said composition is in the form of a powder.

13. A dilute composition according to any one of claims 10 to 12 inclusive which comprises from 0.01 to 2% by weight of active ingredient.

14. A concentrated composition according to any one of claims 10 to 12 inclusive which comprises from 20 to 90% by weight of active ingredient.

15. A process for severely damaging or killing unwanted plants which process comprises applying to said plants, or to the growth medium of said plants, an effective amount of a compound as defined according to any one of claims 1 to 8 inclusive or an effective amount of a composition as defined according to any one

of claims 10 to 14 inclusive.

16. A process for selectively controlling the growth of monocotyledonous weeds in dicotyledonous crops which process comprises applying to said crop, or to the growth medium of said crop, a compound as defined according to any one of claims 1 to 8 inclusive or a composition as defined according to any one of claims 10 to 14 inclusive in an amount sufficient to severely damage or kill the weeds but insufficient to substantially damage the crop.

17. A process according to claim 15 or claim 16 wherein the compound is applied at a rate in the range from 0.05 to 20 kilograms per hectare.

18. A process according to claim 17 wherein the rate is in the range from 0.1 to 10 kilograms per hectare.

19. A process for the synthesis of a compound of formula I as defined according to any one of claims 1 to 8 inclusive which process comprises the condensation, in the presence of an alkaline material, of a 4-(2-pyridylamino)-phenol or -thiophenol of formula IX with a compound of formula X, wherein hal is chlorine, bromine or iodine.

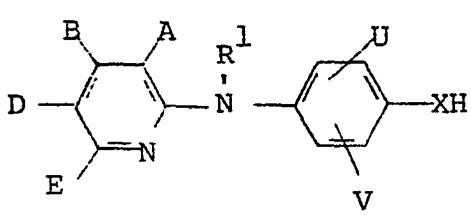

IX

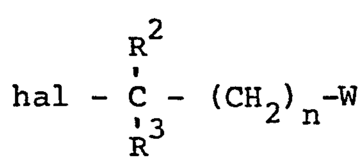

X

20. A process for the synthesis of a compound of formula I as defined according to any one of claims 1 to 8 inclusive which process comprises the condensation

of a pyridine of formula V, wherein L is a leaving group, with an aniline of formula VI.

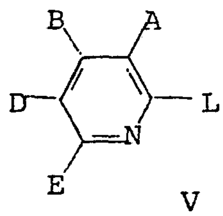

V

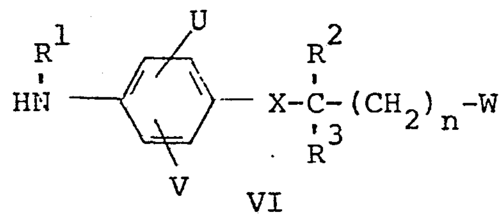

VI

21. A process for the synthesis of a compound of formula I as defined according to any one of claims 1 to 8 inclusive which process comprises the following steps in sequence:

a) the condensation of a pyridine of formula V, wherein L is a leaving group, with an aniline of formula VII, wherein Q is hydroxy, mercapto, $C_1$ to $C_6$ alkoxy, or $C_1$ to $C_6$ alkylthio, to give a compound of formula VIII;

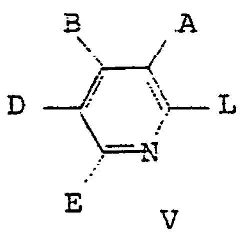

V

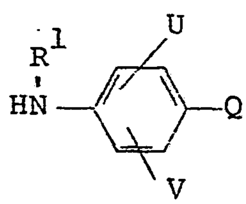

VII

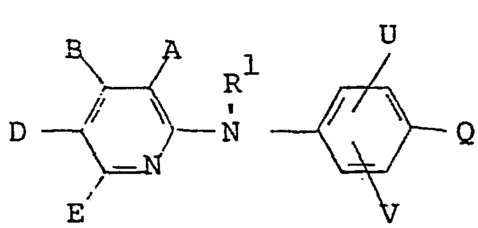

VIII

b) the dealkylation of the compound of formula VIII, wherein Q is $C_1$ to $C_6$ alkoxy or $C_1$ to $C_6$ alkylthio, to give a compound of formula IX; and

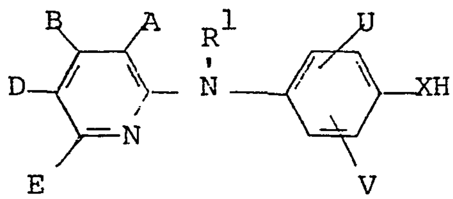

IX

c) the condensation, in the presence of an alkaline material, of a compound of formula IX obtained from step a) or step b) above with a compound of formula X wherein hal is chlorine, bromine or iodine.

$$\text{hal} - \underset{R^3}{\overset{R^2}{C}} - (CH_2)_n\text{-W}$$

X

22. A compound of formula I as defined according to any one of claims 1 to 8 inclusive substantially as herein described with reference to any one of Examples 1 to 12 inclusive.

23. A compound of formula VIII as defined according to claim 9 substantially as herein described with reference to any one of Examples 1 to 7 inclusive, Example 10 or Example 11.

24. A composition as defined according to any one of claims 10 to 14 inclusive substantially as herein described with reference to any one of Examples 13 to 16 inclusive.

25. A process as defined according to any one of claims 19 to 21 inclusive substantially as herein

described with reference to any one of Examples 13 to 16 inclusive.

26. A process as defined according to any one of claims 19 to 21 inclusive substantially as herein described with reference to any one of Examples 1 to 12 inclusive.

DATED this day of 1979

ICI AUSTRALIA LIMITED